# EUROPEAN PATENT APPLICATION

(11) **EP 4 008 342 A1**
(43) Date of publication of application: **08.06.2022**
(21) Application number: 21164128.7
(22) Date of filing: 23.03.2021
(51) Int. Cl.: A61K 39/12, A61P 31/14, A61K 39/09, C07K 16/10, A61K 39/39

(54) **IMMUNOGENIC COMPOSITIONS FOR PRODUCING NEUTRALISING ANTIBODIES AGAINST SARS-COV**

(30) Priority: 03.12.2020 EP 20211707
(71) Applicant: Deutsches Rheuma-Forschungszentrum Berlin, 10117 Berlin (DE)
(72) Inventor: Kruglov, Andrey, 10117 Berlin (DE)
(74) Representative: Hertin und Partner Rechts- und Patentanwälte PartG mbB

(57) **Abstract**

The invention relates to an immunogenic composition for use as a medicament to prevent and/or treat a medical condition associated with a SARS-CoV infection, said composition comprising one or more bacteria of the human microbiota and/or immunogenic parts thereof, wherein said composition is capable of inducing acquired immunity to said infection. The invention further relates to isolated bacteria of the human microbiota that bind to a neutralising antibody that recognises the spike protein of SARS-CoV, preferably the spike protein of SARS-CoV2. In another aspect, the invention relates to an in vitro method for the diagnosis, prognosis, risk stratification and/or therapy management of a human subject with an increased risk of an adverse event associated with a medical condition associated with a SARS-CoV infection (preferably with SARS-CoV-2).

## Description

The present invention is in the field of immunogenic compositions, isolated bacteria and the treatment and/or prevention of medical conditions associated with SARS-CoV infections.

The invention relates to an immunogenic composition for use as a medicament to prevent and/or treat a medical condition associated with a SARS-CoV infection, said composition comprising one or more bacteria of the human microbiota and/or immunogenic parts thereof, wherein said composition is capable of inducing acquired immunity to said infection.

The invention further relates to isolated bacteria of the human microbiota that bind to a neutralising antibody that recognises the spike protein of SARS-CoV, preferably the spike protein of SARS-CoV2.

In another aspect, the invention relates to an in vitro method for the diagnosis, prognosis, risk stratification and/or therapy management of a human subject with an increased risk of an adverse event associated with a medical condition associated with a SARS-CoV infection (preferably with SARS-CoV-2), comprising: providing a sample from the subject, determining the presence and/or absence and/or an amount of one or more bacteria of the human microbiota (gastrointestinal and/or respiratory, preferably oral microbiota) and/or antibodies thereto in said sample, wherein the bacteria binds a neutralising antibody that recognises a spike protein of SARS-CoV (preferably of SARS-CoV-2), wherein the presence and/or absence and/or amount of said one or more bacteria and/or antibodies thereto indicates a likelihood of an adverse event associated with a medical condition associated with a SARS-CoV infection (preferably with SARS-CoV-2).

### BACKGROUND OF THE INVENTION

Human microbiota residing in the respiratory airways, digestive tract and on the skin influences how our immune system functions. Constant interaction between human microbiota and immune system constantly shapes the immune cell repertoire and fitness of the immune system. It is estimated that human microbiota contains more than 3 million genes, thus, the human microbiota may serve as huge reservoir of various antigens. These antigens may mimic proteins of the host as well as other microorganisms and may, therefore, mediate cross-reactive immune responses.

One such example is the development of various autoimmune manifestations via molecular mimicry. For instance, neuromyelitis optica patients have cross-reactive antibodies against commensal microbiota (Ren et al., J Immunol 2012). Recently, a connection between multiple sclerosis (MS) and several commensal bacteria has been reported (Miayuchi et al., Nature 2020). Also, other links between antigens derived from microbiota and development of autoimmunity has been described. Apart from such deleterious functions of microbiota, microbiota also may mediate protection against various infections of bacterial origin with such pathogens as *Citrobacter rodentium, Clostridium difficiles, Pseudomonas aeruginosa* (Robak et al., JCI, 2018) and also of viral origin, like influenza (Ichinohe et al., PNAS, 2011).

Such protection may be mediated by regulating the fitness of the innate immune system, like induction of tonic type I IFN production (Sxhaup et al, Cell 2020), but also by induction of cross-reactive adaptive immune responses (Robak et al., JCI). Interestingly, cross-reactive antibodies targeting gp41 from HIV-1 have been found to be induced by commensal microbiota in the gut (Trama et al., Cell host and microbe, 2014). However, whether such mechanisms occur for other viruses, remains unknown.

Severe acute respiratory syndrome (SARS) is a Coronavirus (CoV) mediated respiratory disease which was first observed in 2002. Based on scientific reports it is assumed that all human CoVs may be of zoonotic origin. Once a human becomes infected, the virus can quickly spread via droplet transmission and close contact between humans, leading to epidemic scenarios or even to a pandemic. As one example, "Coronavirus Disease 2019" (COVID-19) is caused by the pathogenic corona virus SARS-CoV-2. Beginning in December 2019, the virus spread globally within a few weeks and led to an international health emergency. The worldwide pandemic poses huge challenges to health systems and leads additionally to restrictions in social life and weakening of global market economies. Since no effective therapy is available to date, and the disease is associated with high morbidity and mortality, there is a great need for therapeutic or preventative interventions for those who are ill as well as for prophylactic measures to prevent or contain outbreaks. COVID-19 is systemic inflammatory disease caused by a Sars-Cov-2 virus infection of lung and gastrointestinal tract. While a significant proportion of infected people have mild course of disease and can be even asymptomatic, some infected individuals develop severe conditions that require hospitalization in ICU. The reasons for such discrepancy in disease outcome remain poorly understood.

Current treatments rely on the following strategies: vaccination with various constructs containing viral antigens, or inhibition of viral replication machinery. Similarities in protein structure between viruses and commensal microbiota has been described for some viruses. For instance, cross-reactive antibodies against gp41 of HIV-1 and commensal microbiota have been described. And whether these bacteria can induce such antibodies was not addressed (Trama et al.,Cell Host & Microbes, 2014).

To the knowledge of the inventors, there has been no suggestion in the art regarding the ability of human microbiota to induce cross-reactive, neutralizing antibodies against SARS-Cov-2. Furthermore, to the knowledge of the inventors, there has been no suggestion in the art to use an immunogenic composition for preventing or treating a medical condition associated with a SARS-CoV infection, comprising one or more bacteria of the human microbiota and/or immunogenic parts thereof.

### SUMMARY OF THE INVENTION

In light of the prior art the technical problem underlying the present invention is to provide alternative and/or improved means for the treatment and/or prevention of SARS-CoV virus infection and/or medical conditions associated with a SARS-CoV infection.

This problem is solved by the features of the independent claims. Preferred embodiments of the present invention are provided by dependent claims.

The invention therefore relates to an immunogenic composition for use as a medicament to prevent and/or treat a medical condition associated with a SARS-CoV infection, said composition comprising one or more bacteria of the human microbiota and/or immunogenic parts thereof, wherein said composition is capable of inducing acquired immunity to said infection.

In one embodiment, the immunogenic composition for use as described herein, is characterized in that the bacteria is commensal bacteria of the human gastrointestinal, oral and/or respiratory microbiota.

Respiratory infections such as SARS-CoV infection, are of significant clinical and economic importance, as they inflict a substantial health, social and economic burden on people across the world and in particular, in low and lower-middle income countries. Additionally, current therapeutic and prophylactic interventions against respiratory diseases, such as antibiotic treatments, have major constraints, such as the rapid emergence of anti-microbial resistance and disruption of the normal microbiota by use of antibiotics. The present invention therefore provides a surprisingly simple, effective and cost-sensitive approach towards preventing serious disease associated with SARS-CoV infection and means for determining in advance the risk of severe COVID-19 disease.

Without being bound by theory, the present invention is based on the surprising finding that isolated bacteria of the human microbiota induce neutralising antibodies that bind and neutralise SARS-CoV-2 RBD, wherein it is presumed that structural similarity between a bacterial antigen and the Spike protein leads to an immune response in a human subject, producing antibodies against a bacterial antigen, which effectively "cross-reacts" with the RBD of spike protein of SARS CoV, preferably SARS-CoV-2. Cross-immunoreactivity is a phenomenon of structural homology between foreign and self-molecules that can trigger a non-discriminatory immune response. This phenomenon is immunologically possible because of the degeneracy of the immune receptors and the promiscuous mechanisms of antigen recognition.

It is surprising that an immunogenic composition comprising one or more bacteria of the human microbiota and/or immunogenic parts thereof can induce acquired immunity to a SARS-CoV infection. This cross-immunoreactivity enables inhibitory properties of commensal bacteria to combat SARS-CoV and highlights that commensal bacteria can confer protection from SARS-CoV through host-mediated immunity.

It is advantageous that a subject having the commensal bacteria, or having received an administration of such bacteria, as described herein, has already or will acquire a level of immunity against the SARS-CoV infection. This could avoid progression of the disease, or could, in some embodiments, prevent severe progression of disease. As described in greater detail herein, individuals unexposed to SARS-CoV-2 show greatly varying response to infection, some persons responding effectively and avoiding severe disease, whilst others being surprisingly severely debilitated and even leading to death. The immunogenic composition described herein therefore represents a surprising and beneficial approach towards inducing immunity against SARS-COV-2 without exposing individuals to the virus or a vaccine derived from the virus.

It is of great advantage that endogenous, commensal bacteria from the human oral and/or respiratory microbiota may be applied to enhance antibody levels against SARS-CoV-2-Spike-RBD, enabling a reduced risk of severe disease progression.

In a preferred embodiment, the immunogenic composition for use as described herein, is characterized in that the bacteria is of the human oral microbiota.

In some embodiments, the immunogenic composition for use as described herein comprises at least one or more of *Streptococcus salivarius, Bacillus sanfensis, Bacillus pumilis, Bacillus mobilis, Baccilus cereus, Velionella parvulla, Staphylococcus epidermidis* and/or an *Escherichia* strain, *Bifidobacterium pseudocatenulatum, Bifidobacterium longum.*

In another embodiment, the immunogenic composition for use as described herein comprises one or more of *Actinomyces viscosus, Actinomyces naeslundii, Aggregatib, Bacteroides intermediusacter actinomycetemcomitans, Arachnia propionica, Bacteroides spp, Bacteroides gingivalis, Bacteroides melaninogenicus, Bacteroides pneumosintes, Buchnera aphidicola, Campylobacter sputorum, Campylobacter upsaliensis, Candida albicans, Capnocytophaga spp, Citrobacter freundii, Corynebacterium spp, Enterococcus spp, Eubacterium spp, Fusobacterium spp, Fusobacterium nucleatum, Gordonia Bacterium spp, Haemophilus parainfluenzae, Haemophilus paraphrophilus, Lactobacillus spp, Leptotrichia buccalis, Micrococcus spp, Mycoplasma spp, Mycobacterium chelonae, Neisseria spp, Neisseria sicca, Peptococcus spp, Peptostreptococcus spp, Plesiomonas shigelloides, Porphyromonas gingivalis, Rothia dentocariosa, Staphylococcus aureus, Staphylococcus epidermidis, Streptococcus pneumoniae, Streptococcus sobrinus, Streptococcus viridans, Streptococcus salivarius, Torulopsis glabrata, Treponema denticola, Treponema refringens, Veillonella spp, Vibrio sputorum* and/or *Wolinella succinogenes.*

As known in the art, oral commensal nacteria (oral commensals) have been involved in treating disease caused by respiratory pathogens, such as *Streptococcus oralis* and *Streptococcus salivarius,* which can induce protection against middle ear inflammation, which is primarily caused by respiratory pathogens, such as S. *pneumoniae* and *Haemophilus influenzae.* Upon intranasal administration of S. *salivarius* and S. *oralis,* children susceptible to acute otitis media displayed reduced recurrences of disease with no side effects. Furthermore, in a block-randomized challenge trial, 310 healthy individuals (18-25 years) were intranasally inoculated with live N. *lactamica* or sham and the bacterial carriage was monitored for 26 weeks. All those who developed nasopharyngeal colonization by *N. lactamica* revealed a significant reduction in the N. *meningitidis* carriage compared with sham-treated ones. There is therefore support for effective cross-immunoreactivity in the prior art, although no mention has been made until now of the cross-reactivity upon which the present invention is based.

Surprisingly, the binding of ACE2, expressed on the surface of 293T cells, to RBD is inhibited by neutralizing antibodies generated by commensal bacteria as the antibody-inducing agent, not by SARS-CoV-2 infection. Healthy individuals possess anti-RBD IgA at the mucosal surfaces that may therefore inhibit interaction with ACE2. The oral commensal bacteria can therefore be used for treating and/or preventing and/or relieving symptoms of and/or reducing the severity of disease progression of a SARS-CoV infection by inducing an acquired immunity. These oral commensal bacteria are preferably *Streptococcus salivarius, Bacillus sanfensis, Bacillus pumilis, Bacillus mobilis, Baccilus cereus, Velionella parvulla, Staphylococcus epidermidis, Bifidobacterium pseudocatenulatum, Bifidobacterium longum* and/or an *Escherichia* strain. The immunogenic part(s) thereof and/or polypeptide(s) thereof are surprisingly found to be cross-reactive with SARS-CoV infection. In some embodiments, the oral commensal bacteria can also be *Actinomyces viscosus, Actinomyces naeslundii, Aggregatib, Bacteroides intermediusacter actinomycetemcomitans, Arachnia propionica, Bacteroides spp, Bacteroides gingivalis, Bacteroides melaninogenicus, Bacteroides pneumosintes, Buchnera aphidicola, Campylobacter sputorum, Campylobacter upsaliensis, Candida albicans, Capnocytophaga spp, Citrobacter freundii, Corynebacterium spp, Enterococcus spp, Eubacterium spp, Fusobacterium spp, Fusobacterium nucleatum, Gordonia Bacterium spp, Haemophilus parainfluenzae, Haemophilus paraphrophilus, Lactobacillus spp, Leptotrichia buccalis, Micrococcus spp, Mycoplasma spp, Mycobacterium chelonae, Neisseria spp, Neisseria sicca, Peptococcus spp, Peptostreptococcus spp, Plesiomonas shigelloides, Porphyromonas gingivalis, Rothia dentocariosa, Staphylococcus aureus, Staphylococcus epidermidis, Streptococcus pneumoniae, Streptococcus sobrinus, Streptococcus viridans, Streptococcus salivarius, Torulopsis glabrata, Treponema denticola, Treponema refringens, Veillonella spp, Vibrio sputorum* and/or *Wolinella succinogenes.* Immunogenic part(s) thereof and/or polypeptide(s) thereof are surprisingly found to be cross-reactive with SARS-CoV infection.

It is advantageous that treatment and/or prevention of a SARS-CoV infection with human oral commensal bacteria is straightforward and safe. Since commensal bacteria have a long-term biological interaction with humans, they benefit from human colonisation but do not harm the human. This avoids inducing an abnormal immune response in the human body during treatment. Treatment of subjects with commensal bacteria therefore is typically associated with low or no unwanted side effects, as the commensal bacteria are well tolerated by humans, likely the result of (co)evolution in each other's presence. The compositions of the present invention therefore exhibit the additional advantage or exhibiting lower side effects than traditional vaccines.

In one embodiment, the immunogenic composition for use as described herein, is characterized in that the bacteria binds a neutralising antibody that recognises a spike protein of a SARS-CoV.

In a preferred embodiment, the immunogenic composition for use as described herein, is characterized in that the neutralising antibody is an IgA antibody, preferably IgA2.

IgA antibodies at mucosal surfaces are induced upon colonization of the body cavities by commensal microbiota. Apart from induction of IgA, human microbiota at the respiratory airways, digestive tract and on the skin surface influences the fitness of the human immune system. Constant interaction between human microbiota and the immune system shapes the immune cell repertoire and fitness of the immune system. It is estimated that the human microbiota contains several million genes, thus, may serve as a huge reservoir of various antigens. These proteins may partially resemble host proteins, as well as proteins from other microorganisms and may, therefore, mediate cross-reactive immune responses. The production of anti-spike IgA antibodies by the commensal bacteria, according to the invention, is beneficial due to the initial effect of induced IgA antibodies. IgA antibodies, in particular IgA2 antibodies, are typically present at mucosal surfaces and represent an initial bariier to infection by a viral particle.

In some embodiments, the composition is therefore administered to the oral cavity and/or or respiratory system of a subject in order to induce IgA, preferably IgA2, antibodies at the mucosal surface. In some embodiments, such IgA antibodies can be detected using common techniques post-administration of an immunogenic composition of the present invention.

Commensal bacteria enable defence against viral infection through both innate immunity and acquired immunity. When signals from commensal bacteria are abrogated, a potential defect occurs in innate immunity and subsequent adaptive immunity in the lung. Adaptive immunity follows innate immunity and is crucial for specific immunity against respiratory pathogens. Commensal bacteria-mediated adaptive immunity to respiratory pathogens includes both humoral (IgG and IgA) and T cell-mediated responses. For example, rabbit antisera raised against *S. mitis* show cross-reactivity with *S. pneumoniae.* Similar to IgG mediated cross-reactivity, IgA antibodies from the sera, nasal wash, and bronchoalveolar lavage of mice vaccinated with *S. mitis* cross-reacted with *S. pneumoniae* serotypes 2 and 4. It is therefore not only plausible in light of such reports, but also surprising in the context of SARS-CoV infections, that the adaptive immune response directed against SARS-CoV infection is mediated by a neutralising antibody, preferably an IgA antibody, more preferably an IgA2 antibody, induced by commensal bacteria.

Without being bound by theory, one explanation for the IgA production is that early on, before seroconversion in response to SARS-CoV-2 spike protein, activated peripheral B cells may display a type 1 interferon-induced gene expression signature. After seroconversion, activated B cells can lose this signature, and show IL-21- and TGF β -induced gene expression signatures, and express mostly IgG1 and IgA1. In a sustained immune reaction in the patient, activated peripheral B cells may shift to expression of IgA2. This mechanism provides potential support for a preventative use of the composition described herein.

The neutralising antibody, especially the IgA2, are often polyreactive, which may be beneficial for SARS-CoV-2 spike protein reactive antibodies, since the epitopes on spike protein of SARS-CoV-2 mutate extensively during viral evolution.

In one embodiment, the immunogenic composition for use as described herein, is characterized in that the neutralising antibody that recognises a spike protein of a SARS-CoV binds a receptor-binding domain (RBD) sequence according to SEQ ID NO: 1, or to a sequence with at least 70%, preferably at least 80% or 90%, sequence identity to SEQ ID NO: 1 and/or immunogenic fragments thereof.

SEQ ID NO:1 RBD protein:
RVQPTESIVRFPNITNLCPFGEVFNATRFASVYAWNRKRISNCVADYSVLYNSASFSTFKCYGVS
PTKLNDLCFTNVYADSFVIRGDEVRQIAPGQTGKIADYNYKLPDDFTGCVIAWNSNNLDSKVGGN
YNYLYRLFRKSNLKPFERDISTEIYQAGSTPCNGVEGFNCYFPLQSYGFQPTNGVGYQPYRVVV
LSFELLHAPATVCGPKKSTNLVKNKCVNF

In some embodiments, the immunogenic composition as used herein comprises bacteria comprising immunogenic part(s) or antigens, comprising polypeptides with at least 30%, 40%, 50%, 60%, 70%, 80%, 90% to SEQ ID NO:1 and/or immunogenic fragments thereof.

The amino acid sequences of proteins from oral commensal bacteria and the receptor-binding domain (RBD) of spike protein are analysed to identify regions of sequence similarity. The most important finding of this analysis is that some of the similar sequence regions overlap with the receptor-binding domain thought to be an immune target in SARS-CoV infection. It is advantageous that through this surprising finding a targeted therapy using the oral commensal bacteria or immunogenic fragments thereof, can be developed to treat a subject infected with SARS-CoV virus, or prevent infection in a subject.

In another embodiment, the immunogenic composition for use as described herein, is characterized in that the bacteria induces production of a human antibody that recognises the spike protein of a SARS-CoV after administration of said bacteria in a human subject.

In a preferred embodiment, the immunogenic composition for use as described herein, is characterized in that the SARS-CoV is SARS-CoV-2.

Surprisingly, the spike protein of a SARS-CoV-2 can also be recognised by human antibodies, preferably IgA2, which are induced by the commensal bacteria.

It is further surprising that the commensal bacterial as described herein can shape the repertoire of immune cells to respond to SARS-CoV or SARS-CoV-2.

In one embodiment, the immunogenic composition for use as described herein, is characterized in that the acquired immunity lasts for more than 3 months, preferably more than 6 months, more preferably is a lifelong acquired immunity.

The memory B cells formed through human oral microbiota that are cross-reactive with the RBD of the spike protein of SARS-CoV-2 are capable of remembering this pathogen for a faster neutralising antibody production upon future infection. It is advantageous that the acquired immunity triggered by the immunogenic composition as described herein lasts for more than 3 months, preferably more than 6 months, more preferably is a lifelong acquired immunity.

In another embodiment, the immunogenic composition for use as described herein, is characterized in that the medical condition associated with a SARS-CoV infection is selected from the group consisting of respiratory conditions, such as pneumonia, acute respiratory distress or severe acute respiratory syndrome (SARS), viral sepsis, kidney failure, liver failure, disseminated intravascular coagulation (DIC) and pathologic cytokine release syndrome.

In some embodiments, the immunogenic composition for use as described herein, is characterized in that the bacteria have been prepared as a vaccine for administration to a human subject.

In one embodiment, the immunogenic composition for use as described herein, is characterized in that the bacteria have been killed or attenuated, for example by treatment with heat.

In another aspect, the invention relates to isolated bacteria of the human microbiota that bind to a neutralising antibody that recognises the spike protein of SARS-CoV, preferably the spike protein of SARS-CoV2.

In one embodiment, these bacteria are an isolated bacterial population. In one embodiment, these bacteria are isolated by their binding characteristic to an antibody directed against a SARS-CoV spike protein, for example using cell-sorting approaches, such as FACS.

In one embodiment, the isolated bacteria of the human microbiota that bind to a neutralising antibody that recognises the spike protein of SARS-CoV are isolated using this property, for example the bacteria are isolated using an antibody that binds (both) Spike RBD and the bacteria. These bacteria may exist in varying degrees of purity. Such bacteria can be isolated from oral samples, such as saliva samples, for a subject, or from stool samples. These isolated bacteria may then be employed in various applications, such as further study or research of such bacteria to further characterize the relevant antigens, or these bacteria may then be used in preparing compositions of the invention. For example, these bacteria could be cultured, thus increasing their number, before treating and preparing as a vaccine, or other immunogenic composition.

In one embodiment, the isolated bacteria as described herein, is characterized in that the bacteria are commensal bacteria of the human gastrointestinal and/or respiratory microbiota.

In a preferred embodiment, the isolated bacteria as described herein, is characterized in that the bacteria is of the human oral microbiota.

Advantageously, the antibody response to SARS-CoV can be formed by B cells stimulated by the oral microbiota to develop a pre-infection pool of memory B cells cross-reactive with spike protein of SARS-CoV.

In one embodiment, the isolated bacteria as described herein, is characterized in that said bacteria comprise one or more antigens with structural similarity to SEQ ID NO: 1, and/or an immunogenic fragment thereof, wherein said structural similarity is sufficient to induce production of an antibody in a human subject that binds both the bacterial antigen and a protein according to SEQ ID NO: 1.

The structural similarity of bacterial antigens to SEQ ID NO:1 and /or an immunogenic fragment thereof can, in some embodiments, be defined by a sequence similarity or identity on protein level (amino acid sequence identity) to SEQ ID NO 1 or at least 30%, 40%, 50%, 60%, 70%, 80%, 90% or 100%.

In another embodiment, the isolated bacteria as described herein, is characterized in that at least one or more of *Streptococcus salivarius, Bacillus sanfensis, Bacillus pumilis, Bacillus mobilis, Baccilus cereus, Velionella parvulla, Staphylococcus epidermidis, Bifidobacterium pseudocatenulatum, Bifidobacterium longum* or an *Escherichia coli* strains are present.

In another embodiment , the isolated bacteria as described herein, comprise one or more of *Actinomyces viscosus, Actinomyces naeslundii, Aggregatib, Bacteroides intermediusacter actinomycetemcomitans, Arachnia propionica, Bacteroides spp, Bacteroides gingivalis, Bacteroides melaninogenicus, Bacteroides pneumosintes, Buchnera aphidicola, Campylobacter sputorum, Campylobacter upsaliensis, Candida albicans, Capnocytophaga spp, Citrobacter freundii, Corynebacterium spp, Enterococcus spp, Eubacterium spp, Fusobacterium spp, Fusobacterium nucleatum, Gordonia Bacterium spp, Haemophilus parainfluenzae, Haemophilus paraphrophilus, Lactobacillus spp, Leptotrichia buccalis, Micrococcus spp, Mycoplasma spp, Mycobacterium chelonae, Neisseria spp, Neisseria sicca, Peptococcus spp, Peptostreptococcus spp, Plesiomonas shigelloides, Porphyromonas gingivalis, Rothia dentocariosa, Staphylococcus aureus, Staphylococcus epidermidis, Streptococcus pneumoniae, Streptococcus sobrinus, Streptococcus viridans, Streptococcus salivarius, Torulopsis glabrata, Treponema denticola, Treponema refringens, Veillonella spp, Vibrio sputorum* or *Wolinella succinogenes.*

In another aspect, the invention relates to an in vitro method for the diagnosis, prognosis, risk stratification and/or therapy management of a human subject with an increased risk of an adverse event associated with a medical condition associated with a SARS-CoV infection (preferably with SARS-CoV-2), comprising:
a. Providing a sample from the subject,
b. Determining the presence and/or absence and/or an amount of one or more bacteria of the human microbiota (gastrointestinal and/or respiratory, preferably oral microbiota) and/or antibodies thereto in said sample, wherein the bacteria binds a neutralising antibody that recognises a spike protein of SARS-CoV (preferably of SARS-CoV-2), wherein
c. the presence and/or absence and/or amount of said one or more bacteria and/or antibodies thereto indicates a likelihood of an adverse event associated with a medical condition associated with a SARS-CoV infection (preferably with SARS-CoV-2).

In some embodiments, the method as described herein, is characterized in that the absence and/or a low amount of said one or more bacteria and/or antibodies thereto (preferably in comparison to a suitable control, such as from a healthy subject and/or a subject with a SARS-CoV infection but without an elevated risk) indicates an elevated risk of an adverse event and preferably indicates hospital admission, and optionally admission to an intensive care unit (ICU), depending on the status and risk level of the patient.

This aspect of the invention provides a useful measure for medical personnel encountering a patient, who belongs to the coronavirus risk group and/or is infected with coronavirus to assess the possibility of an adverse effect associated with a medical condition associated with a SARS-CoV infection and decide whether hospital admission or other treatment is required. The method is objective and fast, providing a high degree of security to the person in charge of therapeutic measures to make the correct treatment decision.

Determining the presence and/or absence of an amount of human bacteria as described herein can provide information as a biomarker employed in a diagnostic method or in a method for therapy guidance and stratification that may be performed upon first encountering a patient displaying symptoms of Covid-19. It was, as such, a surprising and beneficial discovery that the human bacteria as described herein is a suitable marker indicating an elevated risk of an adverse event and hospital admission, or optionally as a marker for admission to an intensive care unit. It could also predict the number of intensive care beds to be occupied in the near future during the coronavirus pandemic. Such an assessment could also be carried out in addition to other SARS-CoV tests, for example PCR, antigen or antibody tested, as known in the art for e.g. SARS-CoV-2. The method could also regulate the order of vaccination in a population, i.e. by assessing the risk of a subject to a severe SARS-CoV-related disease and thus indicate whether vaccination, either with the compositions described herein or a traditional vaccine, may be required or is to be prioritized.

Embodiments and features of the invention described with respect to the immunogenic composition, the isolated bacteria as well as the in vitro method, are considered to be disclosed with respect to each and every other aspect of the disclosure, such that features characterizing the immunogenic composition, isolated bacteria, or the in vitro method, may be employed to characterize the immunogenic composition, and vice-versa.

The various aspects of the invention are unified by, benefit from, are based on and/or are linked by the common and surprising finding that immunogenic bacteria of the human microbiota and/or immunogenic parts thereof, bind and/or induce production of an antibody directed to a SARS-CoV spike protein, and induce acquired immunity to a SARS-CoV infection when administered to a human subject.

### DETAILED DESCRIPTION OF THE INVENTION

All cited documents of the patent and non-patent literature are hereby incorporated by reference in their entirety.

The present invention relates to an immunogenic composition for use as a medicament to prevent and/or treat a medical condition associated with a SARS-CoV infection, said composition comprising one or more bacteria of the human microbiota and/or immunogenic parts thereof, wherein said composition is capable of inducing acquired immunity to said infection.

As used herein, the term "microbiota" includes bacteria, archaea, protists, fungi and viruses present in an ecological community of commensal, symbiotic and pathogenic microorganisms, as found in and on all multicellular organisms from plants to animals. Microbiota are crucial for immunologic, hormonal and metabolic homeostasis of their host. Different microbiota live on different parts of the body, prefer different foods, and perform different functions. There is an oral microbiota of the mouth, a microbiota of the skin that has many subcategories (the armpits, nose, feet, etc.), and a gut microbiota, among many others.

The term "microbiome" describes either the collective genomes of the microorganisms that reside in an environmental niche or the microorganisms themselves, including microbial structural elements, such as proteins/peptides, lipids, polysaccharides, nucleic acids, mobile genetic elements, or microbial metabolites such as, signalling molecules, toxins, and (an)organic molecules.

The term "microbiota" and the term "microbiome" can be used herein interchangeably.

The term "commensal bacteria" is used as in the art, and typically represents an ensemble of bacterial microorganisms that reside in close proximity and in mutual relation with a host organism.

As used herein, the term "human oral microbiota" shall mean the bacteria and potentially other microorganisms colonized in the oral cavity including in teeth, gingival sulcus, attached gingiva, tongue, cheek, lip, hard palate, and soft palate, further including contiguous with the oral cavity, such as tonsils, pharynx, esophagus, Eustachian tube, middle ear, trachea, lungs, nasal passages, and sinuses. The human oral microbiome is defined as all the microorganisms that are found on or in the human oral cavity and its contiguous extensions. The oral microbiome is comprised of over 600 prevalent taxa at the species level, with distinct subsets predominating at different habitats. The oral microbiota has been extensively characterized by cultivation and culture-independent molecular methods such as 16S rRNA cloning. The Human Oral Microbiome Database (www.homd.org) provides an extensive scope of such microbiota comprising 619 taxa in 13 phyla, such as: *Actinobacteria, Bacteroidetes, Chlamydiae, Chloroflexi, Euryarchaeota, Firmicutes, Fusobacteria, Proteobacteria, Spirochaetes, SR1, Synergistetes, Tenericutes,* and TM7.

As used herein, the term "cross-immunoreactivity" refers to an immune response, such as mediated by an antibody, induced by one antigen but that is also effective against a region of another antigen. For example, such a region can be an epitope which is specific area on the protein structure that is recognized by antibodies or T-cell receptors. Cross-immunoreactivity can occur between epitopes of different domains, or within the same domains, or between epitopes of different viruses or between epitopes of virus and other domains. The "cross-reactivity" of the present invention preferably refers to the production of antibodies that are induced by and bind antigens of commensal bacteria, or other components of the human microbiota, but that bind CARS-CoV-spike protein and preferably neutralise CARS-CoV, in particular SARS-CoV-2.

The innate immune system is the first line of defence against non-self pathogens, which is a non-specific immune response. The innate immune response consists of physical, chemical and cellular defences against pathogens. The main purpose of the innate immune response is to immediately prevent the spread and movement of foreign pathogens throughout the body. The acquired immune system is the second line of defence against non-self pathogens. Adaptive immunity is also referred to as acquired immunity or specific immunity and is only found in vertebrates. The acquired immune response is specific to the pathogen presented. The term "adaptive immune response" and "acquired immune response" are interchangeable.

As used herein, the term "immune receptor" shall mean a receptor, usually on a cell membrane which binds to a substance and causes a response in the immune system, including pattern recognition receptors, Toll-like receptors, killer activated receptors, killer inhibitor receptors, complement receptors, Fc receptors, B cell receptors and T cell receptors.

As used herein, the term "memory B cell" is a sub-type of B cell capable of producing antibodies following primary infection. Memory B cells can survive for decades and repeatedly generate an accelerated and robust antibody-mediated immune response in the case of re-infection. Memory B cells are formed through administration of human oral microbiota, which is cross-reactive with the RBD of the spike protein of SARS-CoV-2, and can remember this pathogen for fast neutralising antibody production upon future infection with a virus with a substantially similar antigen.

As used herein, the term "host-mediated immunity" refers to both innate immunity and acquired immunity which occurs in a host upon viral infection and/or the presence of human microbiota.

As used herein, viral spike protein refers to a transmembrane protein ranging from 1 to 160, or 200, or 250 or 300 or 350 or 400 or 450 or 500 or 600 or 700 or 800 or 900 or 1000 amino acids. Viral spike protein can be highly glycosylated. SARS-CoV diversity is reflected in the variable spike protein, which have evolved into forms differing in their receptor interactions and their response to various environmental triggers of virus-cell membrane fusion. SARS-CoV-2 infects human epithelial cells through interaction with the human ACE2 receptor. A notable distinction between the spike proteins of different coronaviruses is whether it is cleaved or not during assembly and exocytosis of virons. With some exceptions, in most alphacoronaviruses and the betacoronavirus SARS-CoV, the virions harbor a spike protein that is uncleaved, whereas in some beta- and all gamma-coronaviruses the protein is found cleaved between the S1 and S2 domains, typically by furin, a Golgi-resident host protease.

A mutation of SARS-CoV-2 can be caused by mutation of the spike protein of SARS-CoV-2. For example, the spike protein of SARS-CoV-2 acquired a deletion 69-70, deletion 144, N501Y, A570D, D614G, P681H, T716I, S982A or D1118H. Among these, the D614G mutation confers greater infectivity and is now a globally dominant form.

As used herein, the "neutralising antibody" shall mean an antibody defending a target cell from a pathogen or infectious particle by neutralizing any effect it has biologically, preferably leading to stopping or inhibiting a pathogen infecting a target cell. Neutralising a pathogen makes the pathogen no longer infectious or pathogenic. Neutralising antibodies are part of the humoral response of the acquired immune system against viruses, intracellular bacteria and microbial toxins. By binding specifically to RBD protein of SARS-CoV-2 spike protein, neutralising antibodies prevent the virus particle from interacting with its ACE2 receptor of host cell it might infect and destroy.

As used herein, the term "IgA" refers to the IgA class of antibodies. IgA are often referred to as a first line of defence in the resistance against infection. IgA typically inhibits bacterial and viral adhesion to epithelial cells and neutralises bacterial toxins and viruses, both extra- and intracellularly. IgA also eliminates pathogens or antigens via an IgA-mediated excretory pathway where binding to IgA is followed by poly-immunoglobulin receptor-mediated transport of immune complexes. Secretory IgA has an important role in mediating adaptive humoral immune defence at mucosal surfaces, such as gastrointestinal, respiratory and urogenital tracts. Mucosal surfaces are the portal of entry of many pathogens. Secretory IgA produced excessively at mucosal surfaces is the predominant class of Ig found in human external secretions and in tears. In humans, following antigen presentation to T helper cells (Th), and differentiation of Th to Th2, the cytokines IL-10, IL-4, and TGF-β are involved in causing the preferential maturation of B cells into B cells that are committed to producing IgA. In humans there two types of IgA, predominantly IgA1, found in serum and derived in bone marrow, and IgA2, a secretory form of IgA.

The spike protein of SARS-CoV-2 contains a receptor binding domain (RBD) that is particularly important for the infection of target cells. IgA2 antibodies against the spike RBD are detected in the stool of healthy unexposed humans which suggests the presence of IgA2 binding to RBD in unexposed individuals. RBD reactive IgA2 antibodies recognize commensal microbiota. The neutralising anti-RBD SARS-CoV-2 antibodies IgA2 cross-reacts towards commensal bacteria. Thus, the antibody response to SARS-CoV-2 is also shaped by B cells stimulated by microbiota to develop a pre-infection pool of memory B cells cross-reactive with SARS-CoV-2.

In the present invention, the terms "risk stratification" and "therapy management" relate to the grouping of subjects into different risk groups according to their further prognosis and optionally providing suggestions or administration of subsequent therapeutic measures according to a risk group or effectiveness of an ongoing therapy. Risk assessment also relates to stratification for applying preventive and/or therapeutic measures. The term "therapy management" in particular relates to grouping or classifying patients into different groups, such as risk groups or therapy groups that receive certain differential therapeutic measures depending on their classification. The term "therapy management" also relates to grouping or classifying patients with infections or having symptoms of an infectious disease into a group that are not in need to receive certain therapeutic measures.

As used herein, "diagnosis" in the context of the present invention relates to the recognition and (early) detection of a clinical condition of a subject associated with a SARS-CoV infection. Also the assessment of the possibilities of developing adverse symptoms may be encompassed by the term "diagnosis".

"Prognosis" relates to the prediction of an outcome or a specific risk for a subject based on an a clinical condition of a subject linked to a SARS-CoV infection. This may also include an estimation of the chance of recovery or the chance of an adverse outcome for said subject.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of" and "consisting essentially of. Thus, the terms "comprising"/"including"/"having" mean that any further component (or likewise features, integers, steps and the like) can/may be present. The term "consisting of means that no further component (or likewise features, integers, steps and the like) is present.

Methods of isolating and cultivating human commensal bacteria used in the context of the present invention are described in the art. Bacterial isolation can be carried out using a general medium, wherein various bacteria can grow, and selective media that allows growth of specific genera. Examples of general media are nutrient agar (NA), tryptic soy agar (TSA), and brain heart infusion agar (BHIA). Examples of selective media are thiosulfate citrate bile sucrose agar (TCBS) for vibrios, and glutamate starch phenol red agar (GSP) for aeromonads and pseudomonads. By following standard protocols of plating, streaking and re-streaking, bacteria can be isolated. The isolated bacteria can be identified by means of 16S RNA sequencing.

Human serum samples can be obtained from patients infected with Coronavirus and healthy subjects using standard laboratory procedure. Immune mouse serum against human commensal bacteria was produced by repeated immunization of 6-8-week old mice. The method is known in the art. The reactivity of the spike protein with anti-serum can be determined by means of a number of methods known in the art, for example ELISA, latex agglutination assay, western blot and immunoprecipitation.

### Medical Indications:

In one aspect of the present invention there is provided a composition as herein described for use in the treatment and/or prevention of a viral infection in a subject and/or a medical condition associated with a viral infection. Preferred viral infections to be treated or associated diseases are those described herein.

In one aspect of the present invention there is provided a immunogenic composition or combination according to the invention as described herein for use in the treatment of a medical condition associated with a SARS Coronavirus, wherein the medical condition associated with a SARS Coronavirus is preferably COVID-19 or a SARS Coronavirus-associated respiratory disease.

As used herein, the "patient" or "subject" may be a vertebrate. In the context of the present invention, the term "subject" includes both humans and animals, particularly mammals, and other organisms.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

In one embodiment, the treatment described herein relates to either reducing or inhibiting Coronavirus infection or symptoms thereof via preventing viral entry into target cells.

The prophylactic therapy as described herein is intended to encompass prevention or reduction of risk of Coronavirus infection, due to a reduced likelihood of Coronavirus infection of cells via interaction with the ACE2 protein after treatment with the agents described herein.

In one embodiment, a patient with symptoms of an infectious disease may be treated.

A "patient with symptoms of an infectious disease" is a subject who presents with one or more of, without limitation, fever, diarrhea, fatigue, muscle aches, coughing, if have been bitten by an animal, having trouble breathing, severe headache with fever, rash or swelling, unexplained or prolonged fever or vision problems. Other symptoms may be fever and chills, very low body temperature, decreased output of urine (oliguria), rapid pulse, rapid breathing, nausea and vomiting. In preferred embodiments the symptoms of an infectious disease are fever, diarrhea, fatigue, muscle aches, rapid pulse, rapid breathing, nausea and vomiting and/or coughing.

In one embodiment, a patient with symptoms of a viral infection of the respiratory tract may be treated.

As used herein, a patient with "symptoms of a viral infection of the respiratory tract" is a subject who presents with one or more of, without limitation, cold-like symptoms or flu-like illnesses, such as fever, cough, runny nose, sneezing, sore throat, having trouble breathing, headache, muscle aches, fatigue, rapid pulse, rapid breathing, nausea and vomiting, lack of taste and/or smell and/or malaise (feeling unwell).

In some embodiments, symptoms of infection with a SARS-virus are fever, sore throat, cough, myalgia or fatigue, and in some embodiments, additionally, sputum production, headache, hemoptysis and/or diarrhea. In some embodiments, symptoms of an infection with a SARS-coronavirus, for example SARS-CoV-2, are fever, sore throat, cough, lack of taste and/or smell, shortness of breath and/or fatigue.

In one embodiment, a patient that is at risk of developing a severe acute respiratory syndrome (SARS) may be treated.

As used herein, the term "a patient that is at risk of developing a severe acute respiratory syndrome (SARS)" relates to a subject, preferably distinct from any given person in the general population, who has an increased (e.g. above-average) risk of developing SARS. In some embodiments, the patient has symptoms of SARS or symptoms of a SARS Coronavirus infection. In some embodiments, the patient has no symptoms of SARS or symptoms of a SARS Coronavirus infection. In some embodiments, the subject has been in contact with people with SARS Coronavirus infections or symptoms. In some embodiments, the person at risk of developing SARS has been tested for the presence of a SARS Coronavirus infection. In some embodiments, the person at risk of developing SARS has tested positive for the presence of a SARS Coronavirus infection, preferably a coronavirus infection.

In embodiments, the patient at risk of developing SARS is an asymptomatic patient that shows no specific symptoms of SARS (yet). An asymptomatic patient may be at risk of developing SARS because the patient has been in contact with a person infected with a SARS Coronavirus. For example, the asymptomatic patient may have been identified as being at risk of developing SARS by a software application (app) that is installed on his smart phone or corresponding (portable) device and that indicates physical proximity or short physical distance to an infected patient that uses a corresponding app on its respective mobile device/smart phone. Other methods of determining contact/physical proximity to an infected person are known to the skilled person and equally apply to the method of the invention.

In some embodiments, the patient that has or is at risk of developing a severe acute respiratory syndrome (SARS) has a coronavirus infection.

Coronaviruses are a group of related viruses that cause diseases in mammals and birds. The scientific name for coronavirus is *Orthocoronavirinae* or *Coronavirinae.* Coronavirus belongs to the family of *Coronaviridae.* The family is divided into *Coronavirinae* and *Torovirinae* sub-families, which are further divided into six genera: *Alphacoronavirus, Betacoronavirus, Gammacoronavirus, Deltacoronavirus, Torovirus,* and *Bafinivirus.* While viruses in the genera *Alphacoronaviruses* and *Betacoronaviruses* infect mostly mammals, the *Gammacoronavirus* infect avian species and members of the *Deltacoronavirus* genus have been found in both mammalian and avian hosts.

In humans, coronaviruses cause respiratory tract infections that can be mild, such as some cases of the common cold, and others that can be lethal, such as SARS, MERS, and COVID-19. Coronaviruses are enveloped viruses with a positive-sense single-stranded RNA genome and a nucleocapsid of helical symmetry. The genome size of coronaviruses ranges from approximately 27 to 34 kilobases, the largest among known RNA viruses.

Various species of human coronaviruses are known, such as, without limitation, Human coronavirus OC43 (HCoV-OC43), of the genus β-CoV, Human coronavirus HKU1 (HCoV-HKU1), of the genus β-CoV, Human coronavirus 229E (HCoV-229E), α-CoV, Human coronavirus NL63 (HCoV-NL63), α-CoV, Middle East respiratory syndrome-related coronavirus (MERS-CoV), Severe acute respiratory syndrome coronavirus (SARS-CoV), Severe acute respiratory syndrome coronavirus 2 (SARS-CoV-2).

Coronaviruses vary significantly in risk factor. Some can kill more than 30% of those infected (such as MERS-CoV), and some are relatively harmless, such as the common cold. Coronaviruses cause colds with major symptoms, such as fever, and a sore throat, e.g. from swollen adenoids, occurring primarily in the winter and early spring seasons. Coronaviruses can cause pneumonia (either direct viral pneumonia or secondary bacterial pneumonia) and bronchitis (either direct viral bronchitis or secondary bacterial bronchitis). Coronaviruses can also cause SARS.

Advances in nucleic acid sequencing technology (commonly termed Next-Generation Sequencing, NGS) are providing large sets of sequence data obtained from a variety of biological samples and allowing the characterization of both known and novel virus strains. Established methods are therefore available for determining a Coronavirus infection.

### Compositions and administration:

As used herein, the "respiratory tract" is commonly considered to comprise the nose, pharynx, larynx, trachea, and the lung with its different compartments. The respiratory tract is involved with the process of respiration in mammals, and is a part of the respiratory system, and is lined with respiratory mucosa or respiratory epithelium.

As used herein, the "immunogenic composition" refers to a foreign substance, such as an antigen, to provoke an immune response in the body of a human or other animal. In other words, immunogenicity is the ability to induce a humoral and/or cell-mediated immune responses.

As used herein, the "vaccine" refers to a biological preparation that provides active acquired immunity to a particular infectious disease. A vaccine typically contains an agent that resembles a disease-causing microorganism and is often made from weakened or killed forms of the microbe, its toxins, or one of its surface proteins, or with nucleic acid molecules encoding a relevant antigen. The agent stimulates the body's immune system to recognize the agent as a threat, destroy it, and to further recognize and destroy any of the microorganisms associated with that agent that it may encounter in the future. Vaccines can be prophylactic to prevent or ameliorate the effects of a future infection by a natural or "wild" pathogen, or therapeutic to fight a disease that has already occurred, such as cancer.

An immunogenic polypeptide from microbiome of the invention can also be used to prepare an immunogenic composition (e.g., a vaccine) for generating antibodies against coronavirus (e.g., SARS CoV) in a subject susceptible to the coronavirus. Such compositions can be prepared, e.g., according to the method described in the examples below, or by any other equivalent methods known in the art. The composition contains an effective amount of an immunogenic composition of the invention, and a pharmaceutically acceptable carrier. The carrier is selected on the basis of the mode and route of administration, and standard pharmaceutical practice. Suitable pharmaceutical carriers and diluents, as well as pharmaceutical necessities for their use, are described in Remington's Pharmaceutical Sciences. An adjuvant, e.g., a cholera toxin, Escherichia coli heat-labile enterotoxin (LT), liposome, immune-stimulating complex (ISCOM), or immunostimulatory sequences oligodeoxynucleotides (ISS-ODN), can also be included in a composition of the invention, if necessary. Methods for preparing vaccines are generally well known in the art, as exemplified by U.S. Pat. Nos. 4,601,903; 4,599,231; 4,599,230; and 4,596,792. Vaccines may be prepared as injectables, as liquid solutions or emulsions.

Excipients in a composition may include water, saline, dextrose, glycerol, ethanol, and combinations thereof. The vaccine may further contain minor amounts of auxiliary substances such as wetting or emulsifying agents, pH buffering agents, or adjuvants to enhance the effectiveness of the vaccines. Methods of achieving adjuvant effect for the vaccine includes use of agents, such as aluminum hydroxide or phosphate (alum), commonly used as 0.05 to 0.1 percent solutions in phosphate buffered saline. Vaccines may be administered parenterally, by injection subcutaneously or intramuscularly. Alternatively, other modes of administration including suppositories and oral formulations may be desirable. For suppositories, binders and carriers may include, for example, polyalkalene glycols or triglycerides. Oral formulations may include normally employed excipients such as, for example, pharmaceutical grades of saccharine, cellulose, magnesium carbonate and the like. These compositions take the form of solutions, suspensions, tablets, pills, capsules, sustained release formulations or powders and contain 10- 95% of the S protein, fragment analogs, or peptides. Inhalation means may also be used to administer the composition, for example inhalation devices may be used to administer the bacteria, for example as a spray, diffusion, dispersion, or powder formulation, to the respiratory mucosa, where sufficient immunogenic response can be generated.

The vaccines are administered in a manner compatible with the dosage formulation, and in an amount that is therapeutically effective, protective and immunogenic. The quantity to be administered depends on the subject to be treated, including, for example, the capacity of the individual's immune system to synthesize antibodies, and if needed, to produce a cell-mediated immune response. Precise amounts of active ingredient required to be administered depend on the judgment of the practitioner. However, suitable dosage ranges are readily determinable by one skilled in the art and may be of the order of micrograms of the polypeptide of this invention. Suitable regimes for initial administration and booster doses are also variable, but may include an initial administration followed by subsequent administrations. The dosage of the vaccine may also depend on the route of administration and varies according to the size of the host. A subject susceptible to coronavirus infection can be identified and administered a polypeptide-containing composition of the invention. The dose of the composition depends, for example, on the particular polypeptide, whether an adjuvant is co-administered with the polypeptide, the type of adjuvant co-administered, the mode and frequency of administration, as can be determined by one skilled in the art.

Also within the scope of this invention is a diagnostic or prognostic method using the above described microbiota or antibodies. Presence of the polypeptides or antibodies in a subject indicates that the subject is infected with a coronavirus or has a sufficient cross-reactivity. The presence of antibodies against spike protein or microbiota described herein may also represent the presence of a pre-existing immunity in the subject to a SARS-CoV infection, even if the subject has not yet been infected. To detect the antibodies or microbiota, one can obtain a test sample from a subject and detect the presence or absence of the antibodies or microbiota using standard techniques, including PCR, ELISAs, immunoprecipitations, immunofluorescence, EIA, RIA, and Western blotting analysis.

As used herein, the "structural similarity" refers to the similarity of proteins' three-dimensional structure. Since a protein's amino acid sequence determines 3D structure, which in turn determines protein function, sequence similarity is also a good predictor of functional similarity. However, at times, slightly different amino acid sequences can yield very different structures, and similar sequences can sometimes yield dissimilar structures. The structural similarity between two proteins is preferably measured by the root-mean-square-deviation (RMSD) in their best-superimposed atomic coordinates. RMSD is the golden rule of measuring structural similarity when the structures are nearly identical; it, however, fails to detect the higher order topological similarities in proteins evolved into different shapes. The structural similarity between two proteins could also be determined by computational techniques such as, ab initio-techniques, homology modelling, threading as well as experimental techniques for structure determination, such as, X-ray Diffraction Crystallography, Nuclear Magnetic Resonance Spectroscopy, etc. According to embodiments of the invention, the bacteria comprise one or more antigens with structural similarity to SEQ ID NO: 1, and/or an immunogenic fragment thereof, wherein said structural similarity is sufficient to induce production of an antibody in a human subject that binds both the bacterial antigen and a protein according to SEQ ID NO: 1. The structural similarity can thus be functionally assessed without undue effort, using the techniques disclosed in the examples herein.

### FIGURES

The following figures are presented to describe particular embodiments of the invention, without being limiting in scope.

### Brief description of the Figures:

Figure 1. Presence of IgA antibodies reactive against RBD in healthy individuals.
Figure 2. Neutralizing anti-RBD antibodies recognize commensal bacteria.
Figure 3. Identification of bacteria recognized by neutralizing anti-RBD antibodies.
Figure 4. Distinct microbiota species can induce neutralizing anti-RBD response
Figure 5. Oral microbiota changes during COVID-19.
Figure 6. Fecal microbiota changes during severe COVID-19.
Fig. 7: Binding of bacteria by human and rabbit anti-RBD antibodies.
Fig. 8: Isolated bacterial strains bound by neutralizing anti-RBD antibodies.
Fig. 9: Immunisation with single bacteria strains induced anti-RBD Ig response.
Fig. 10: Commercially available probiotic Streptococcus salivarius K12 is recognized by anti-RBD antibodies.

### Detailed description of the Figures:

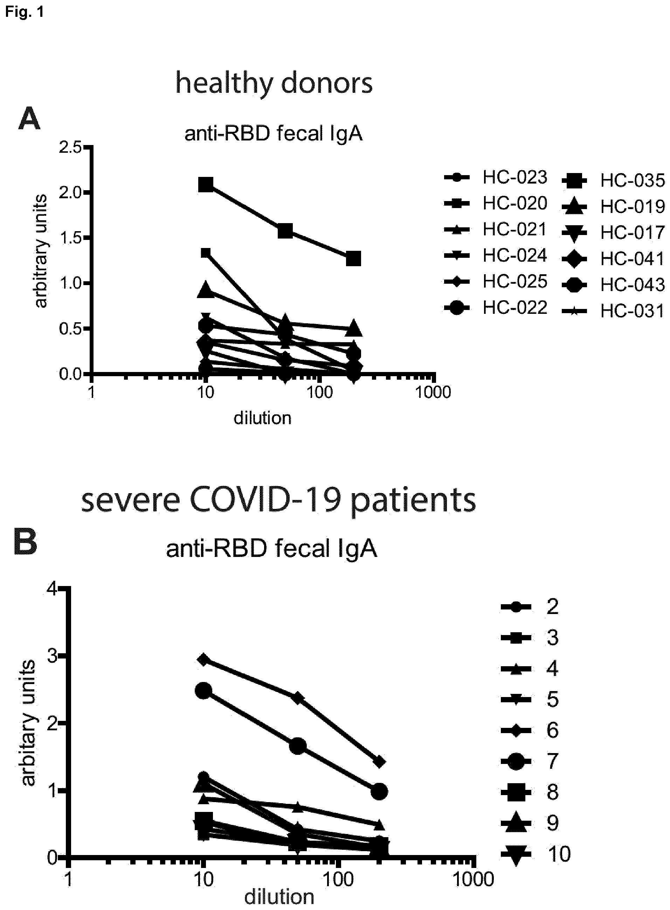

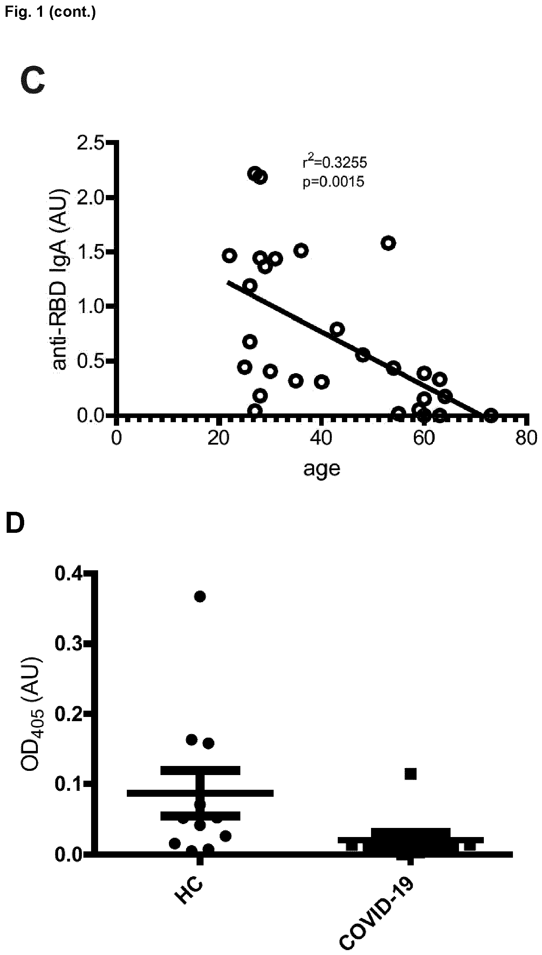

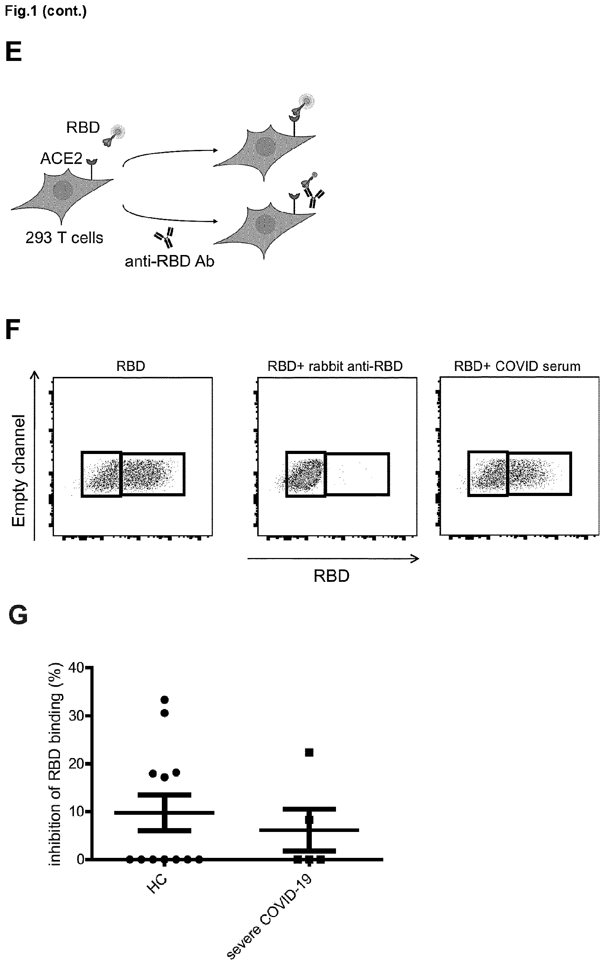

**Figure 1****. Presence of IgA antibodies reactive against RBD in healthy individuals.** Levels of anti-RBD IgA in fecal supernatants of healthy (A) and severe COVID-19 (B) individuals. (C) Correlation of the levels of anti-RBD IgA with the age in healthy individuals. (D) Presence of anti-RBD IgA2 in purified IgA fraction from healthy and severe COVID-19 individuals. (E). Scheme of flow cytometry assay for the analysis of inhibition of ACE2-RBD binding by antibodies. (F). Representative dot plots of ACE2 expressing 293 T cells incubated with fluorescently labelled RBD alone or in the presence of neutralizing antibodies or serum from COVID-19 patient. (G). Inhibition of RBD binding to ACE2 by IgA purified from feces of healthy people and severe COVID-19 patients.

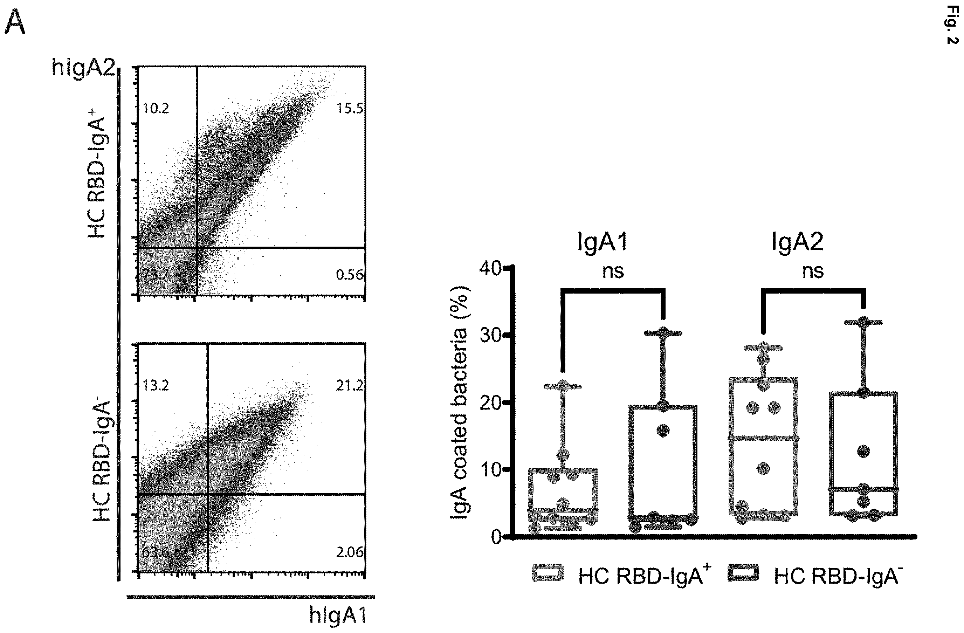

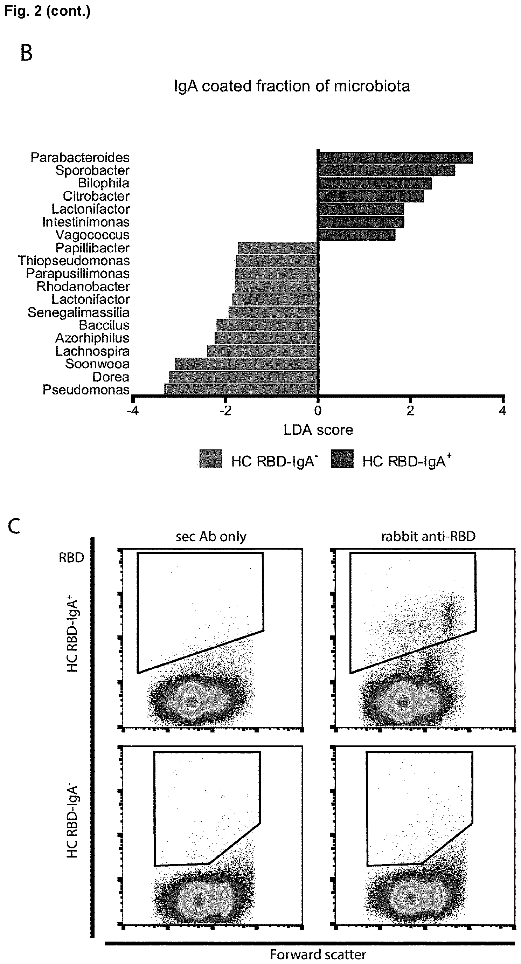

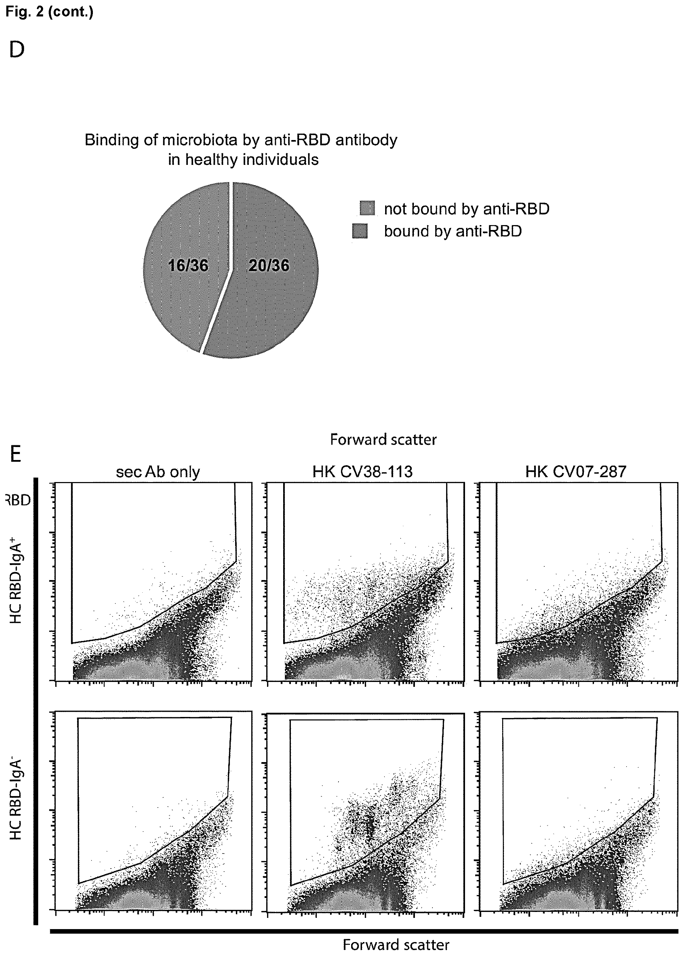

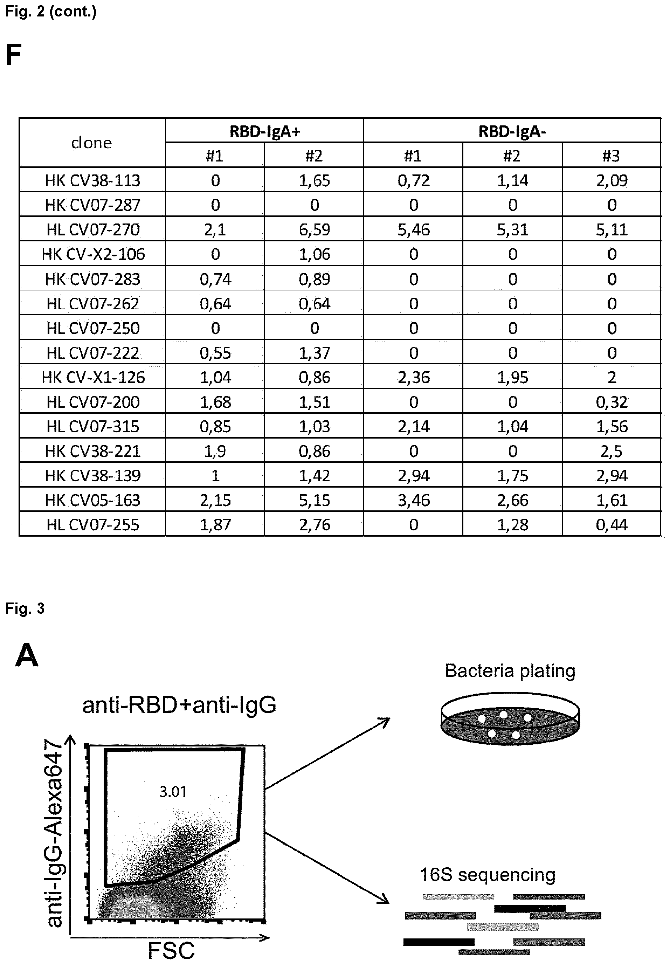

**Figure 2****. Neutralizing anti-RBD antibodies recognize commensal bacteria.** A. Linear discriminant analysis (LDA) scores of whole stool microbiota (A) and IgA bound fraction (B) isolated from healthy individuals that have anti-RBD IgA (HC RBD-IgA+) and lack anti-RBD IgA (HC RBD-IgA-). (C) Representative dot plots of human microbiota stained with neutralizing anti-RBD antibody raised in rabbit. (D) Frequency of healthy individuals harboring bacteria reactive towards rabbit neutralizing anti-RBD antibody. (D) Representative dot plots of microbiota stained with human neutralizing anti-RBD antibodies cloned from COVID-19 patients. E. Binding of human neutralizing anti-RBD antibodies towards microbiota from healthy individuals. E. Binding of distinct and similar bacteria populations by human and rabbit anti-RBD antibody.

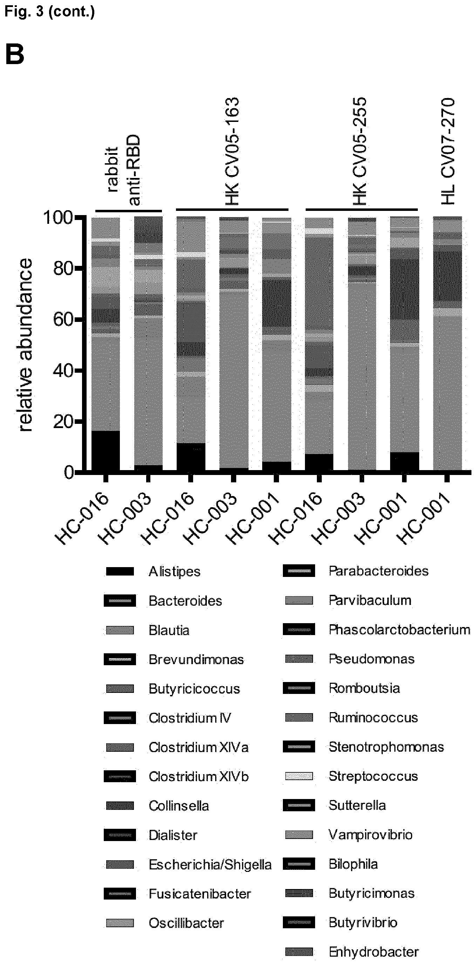

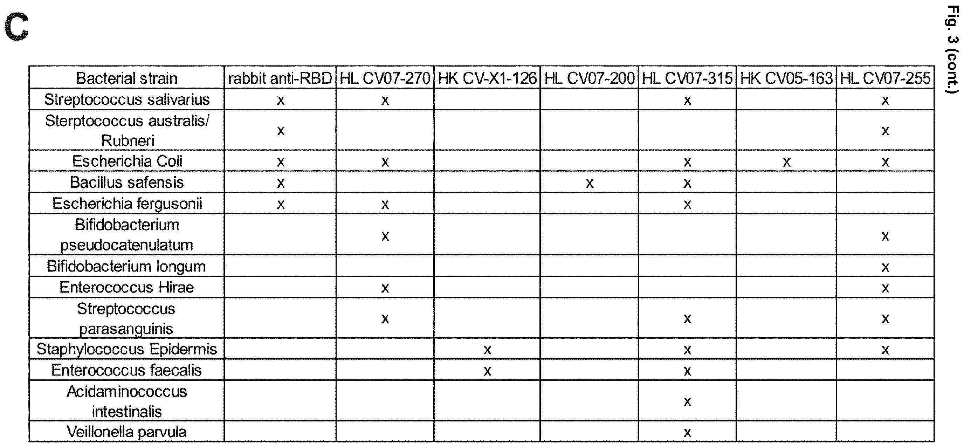

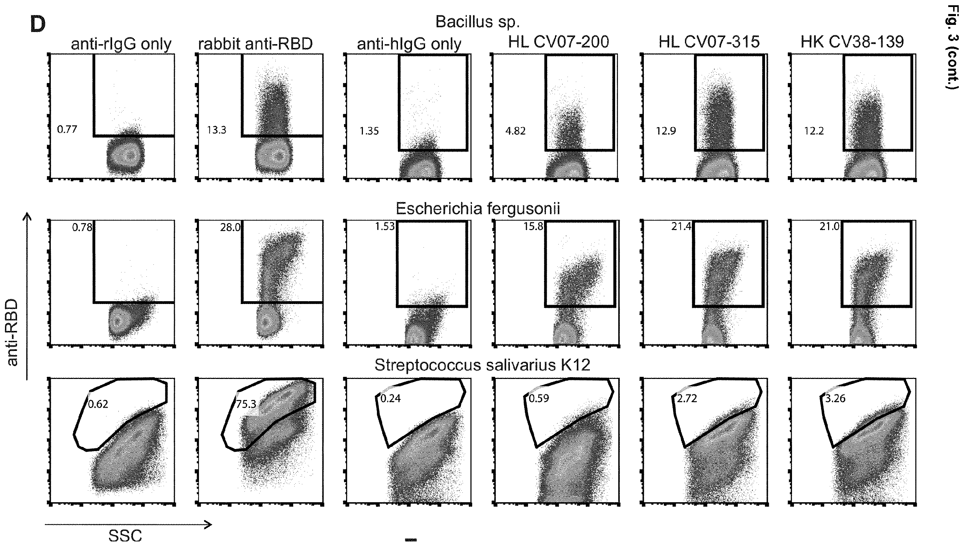

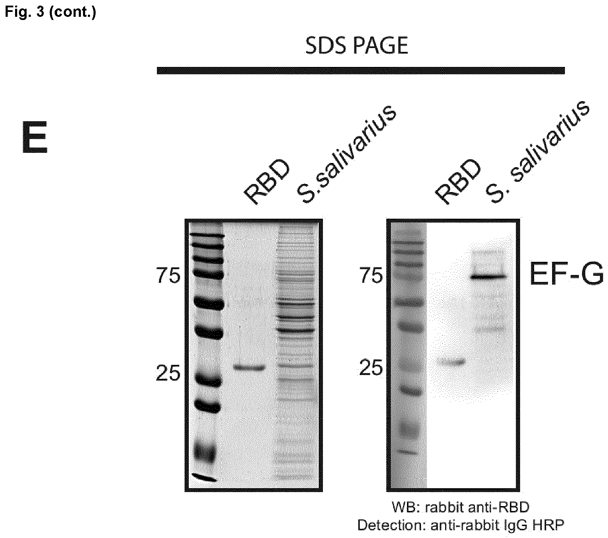

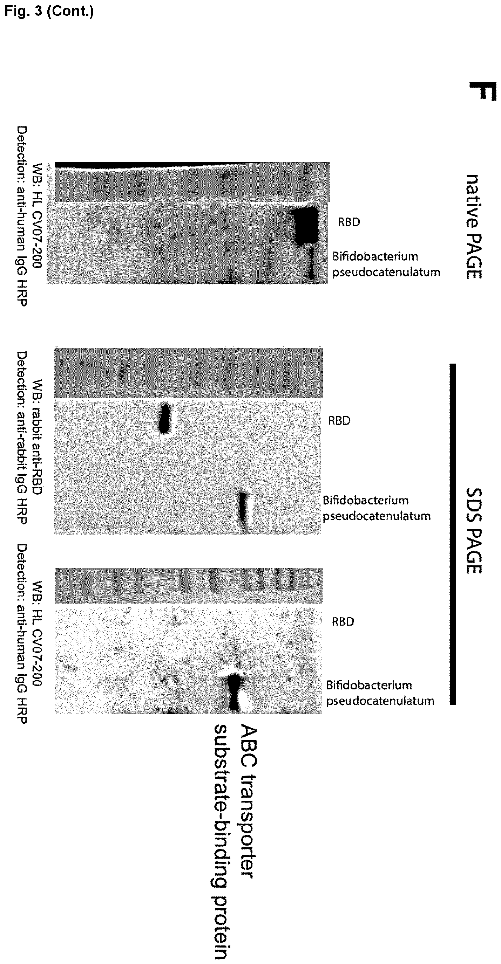

**Figure 3****. Identification of bacteria recognized by neutralizing anti-RBD antibodies.** (A) Strategy for the identification of bacteria that is bound by anti-RBD antibodies. (B) Relative abundance of bacterial genera of greater than 1% abundance in sorted bacterial fractions bound by various anti-RBD antibodies. (C) List of cloned bacteria isolated based on the binding to anti-RBD antibodies. (D) Binding of distinct anti-RBD antibodies to isolated bacteria. (E). Western blot analysis of rabbit anti-RBD binding to Streptococcus salivarius lysate. (F). Western blot analysis of rabbit anti-RBD and HL CV07-200 binding to Bifidobacterium pseudocatenulatum lysate.

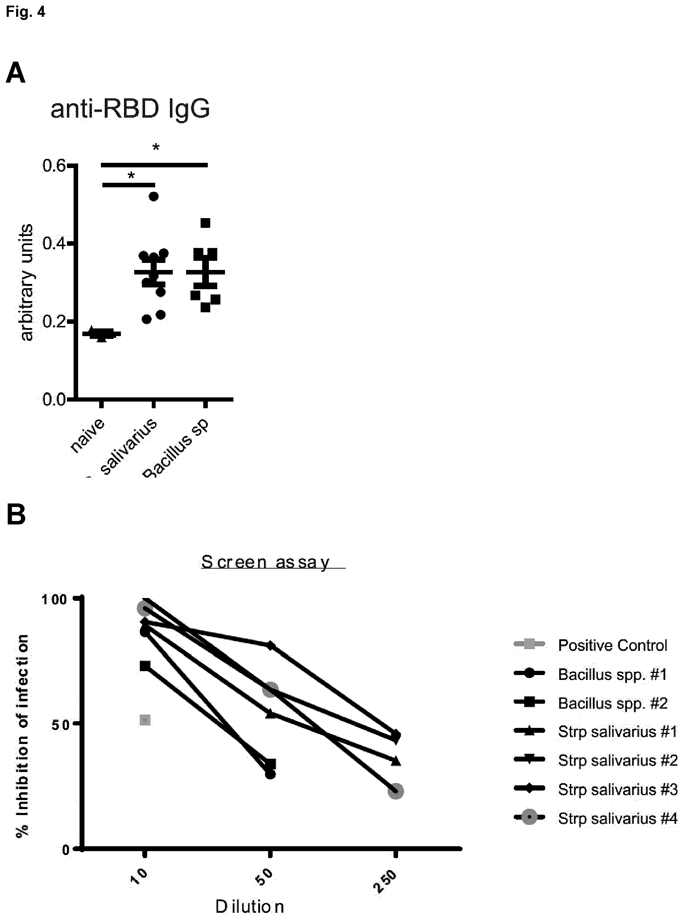

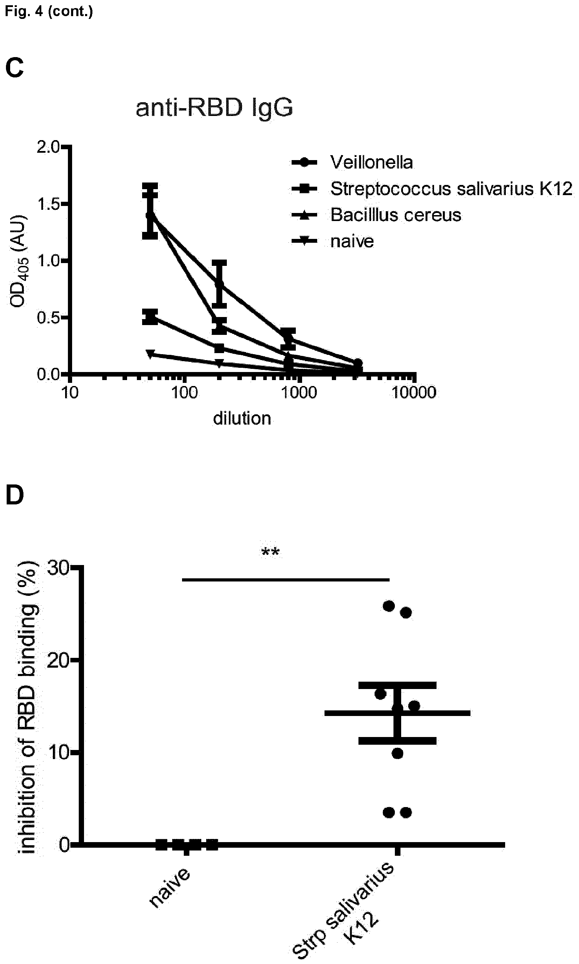

**Figure 4****. Distinct microbiota species can induce neutralizing anti-RBD response** (A) anti-RBD IgG titers at day 14 in mice immunized with heat-killed bacteria. (B) Neutralization of virus by sera from mice immunized with heat-killed bacteria (day 14 after immunization). (C) anti-RBD IgG response in sera of immunized mice 2 months after initial challenge. (D) Inhibition of RBD binding to ACE2 by sera from streptococcus salivarius K12 immunized animals.

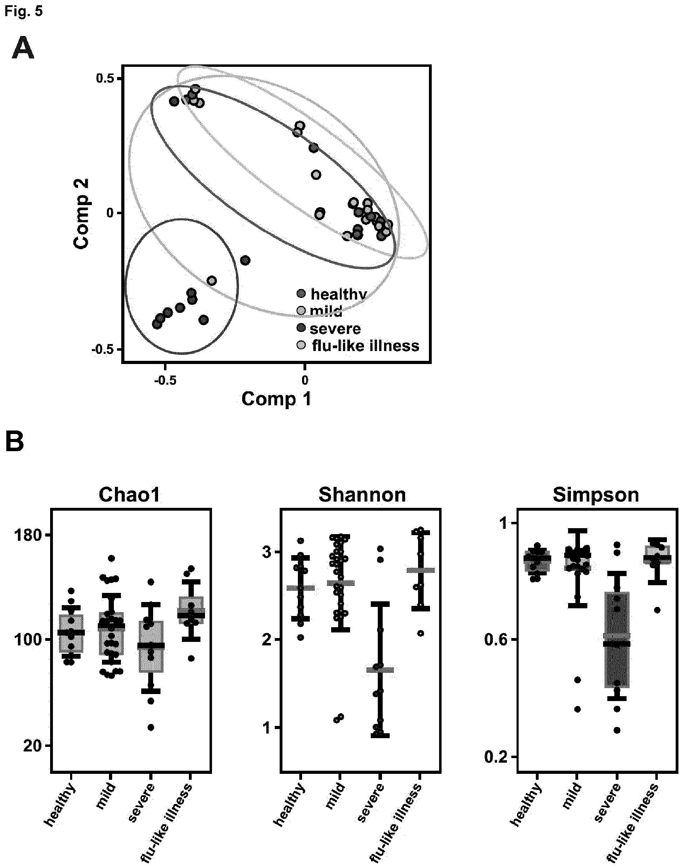

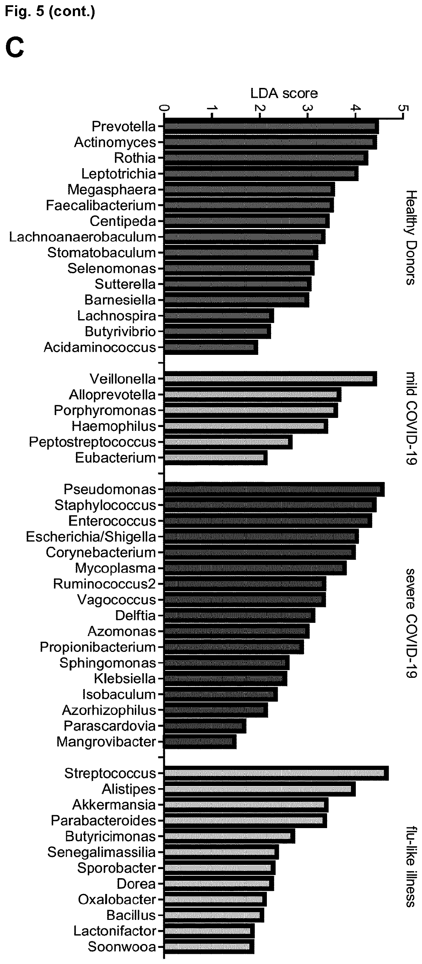

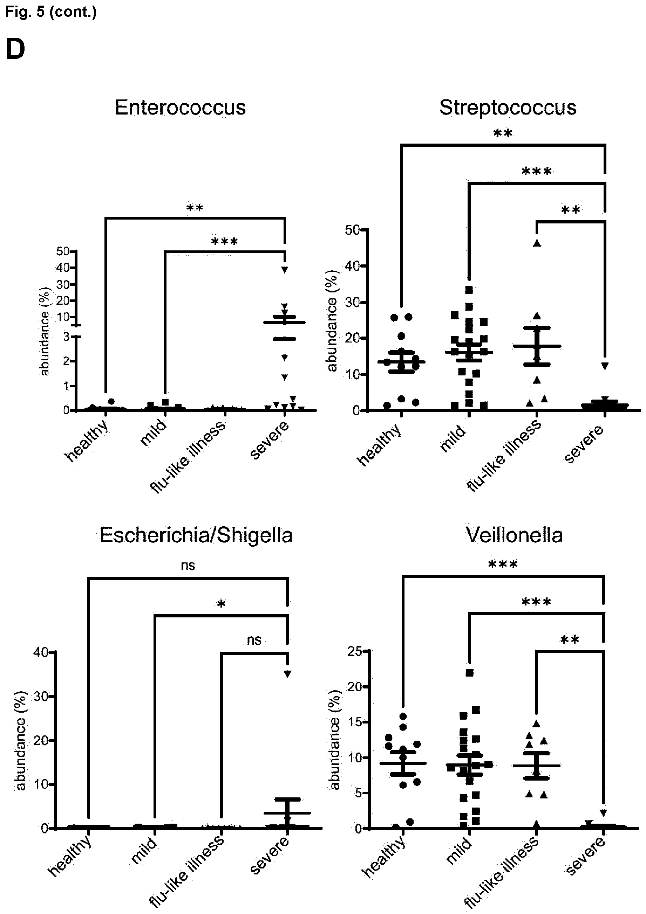

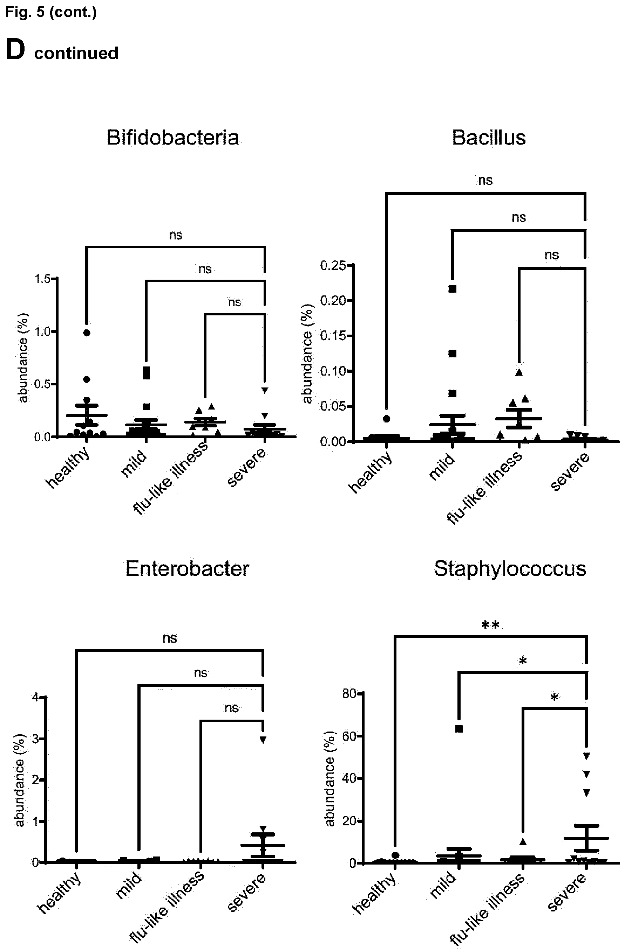

**Figure 5****. Oral microbiota changes during COVID-19.** (A) Principal Component analysis of microbiota of swabs collected from healthy, mild and severe COVID-19 patients and patients with flu-like illness. (B) Microbial diversity of oral microbiota collected by swabbing of healthy, mild and severe COVID-19 patients and patients with flu-like illness. (C) LDA scores of genera between healthy, mild and severe COVID-19 and flu-like illness. (D) Abundance of selected bacterial genera in swabs from healthy, mild and severe COVID-19 and flu-like illness. Statistical analysis is one-way ANOVA.

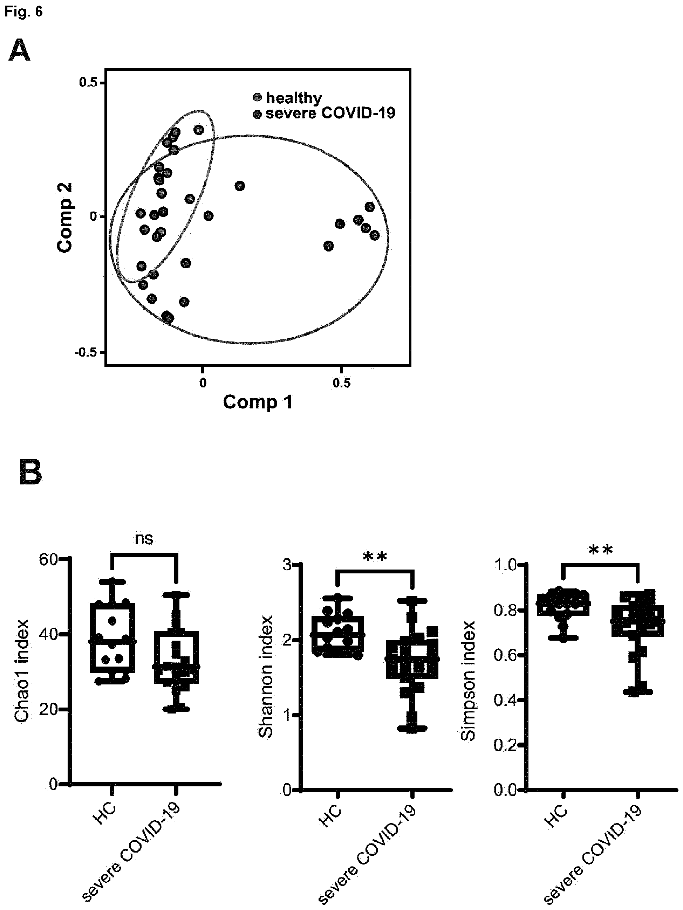

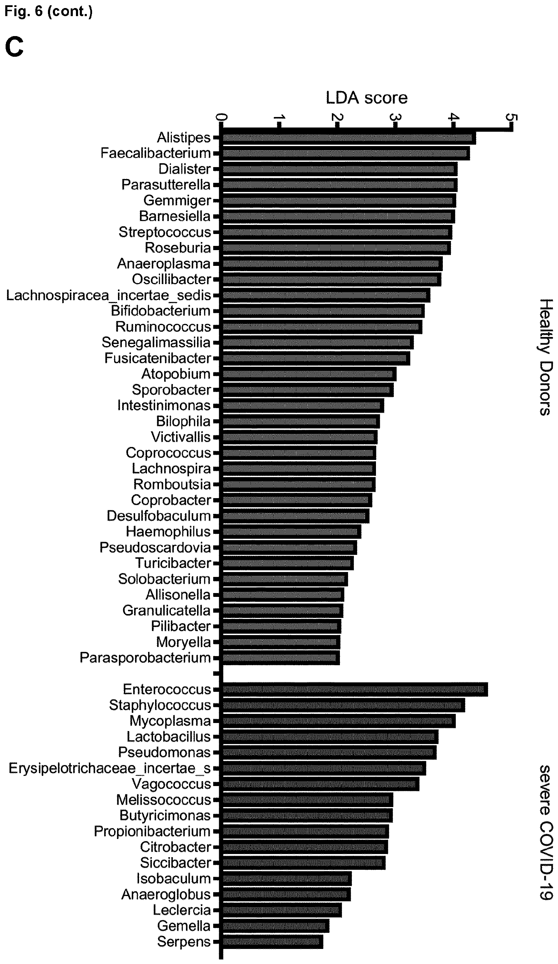

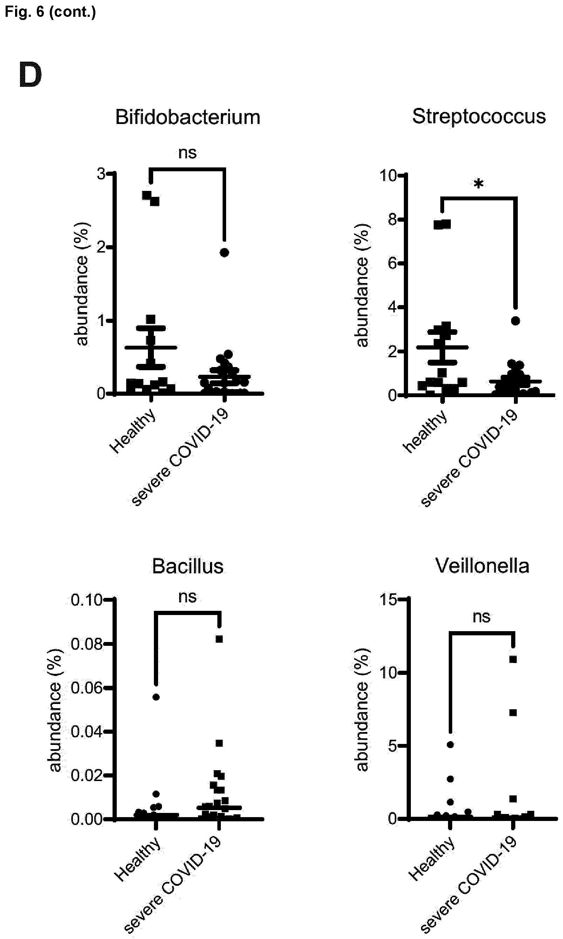

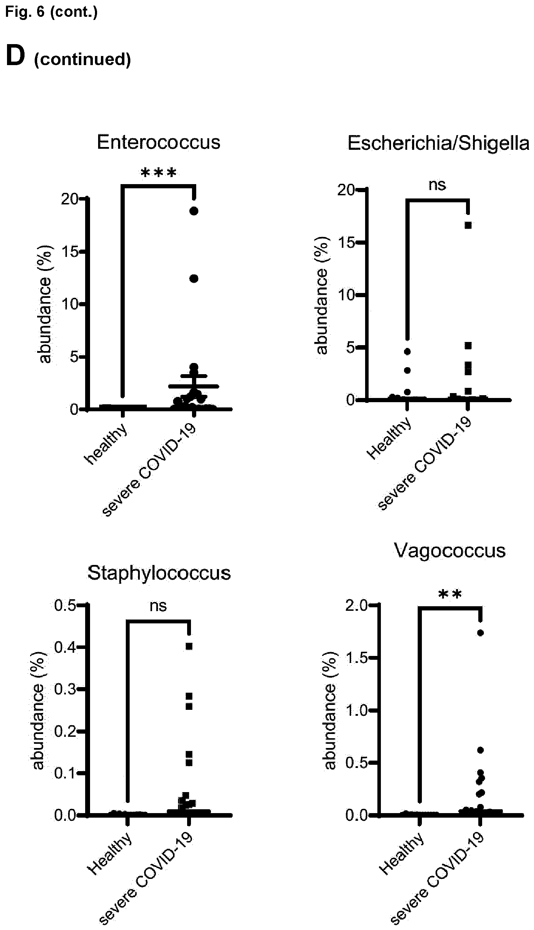

**Figure 6****. Fecal microbiota changes during severe COVID-19.** (A) Principal Component analysis of microbiota of stools collected from healthy and severe COVID-19 patients. (B) Microbial diversity of fecal microbiota of healthy and severe COVID-19 patients. (C) LDA scores of genera between fecal microbiota in healthy and severe COVID-19. (D) Abundance of selected bacterial genera in feces from healthy and severe COVID-19. Statistical analysis is one-way ANOVA.

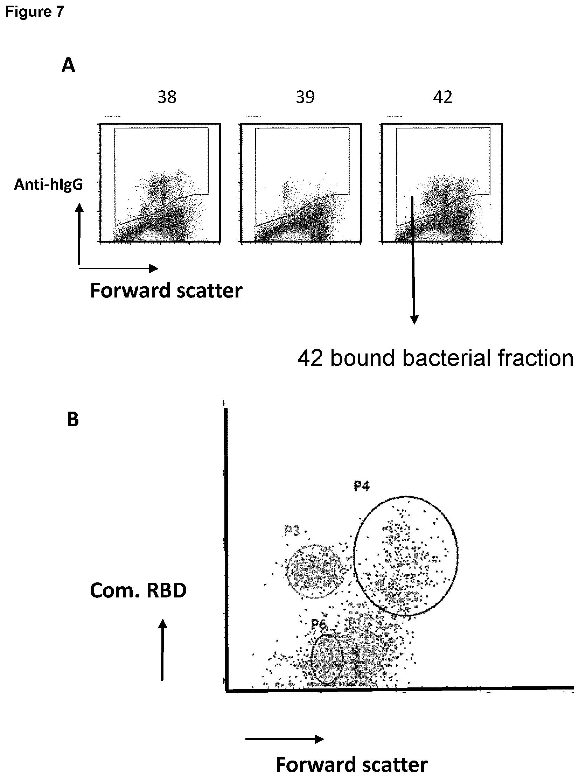

**Fig. 7****: Binding of bacteria by human and rabbit anti-RBD antibodies.** (A) Staining of microbiota by human anti-RBD antibodies;(B) Antibody #42 recognises similar bacteria populations to commercial neutralizing antibody

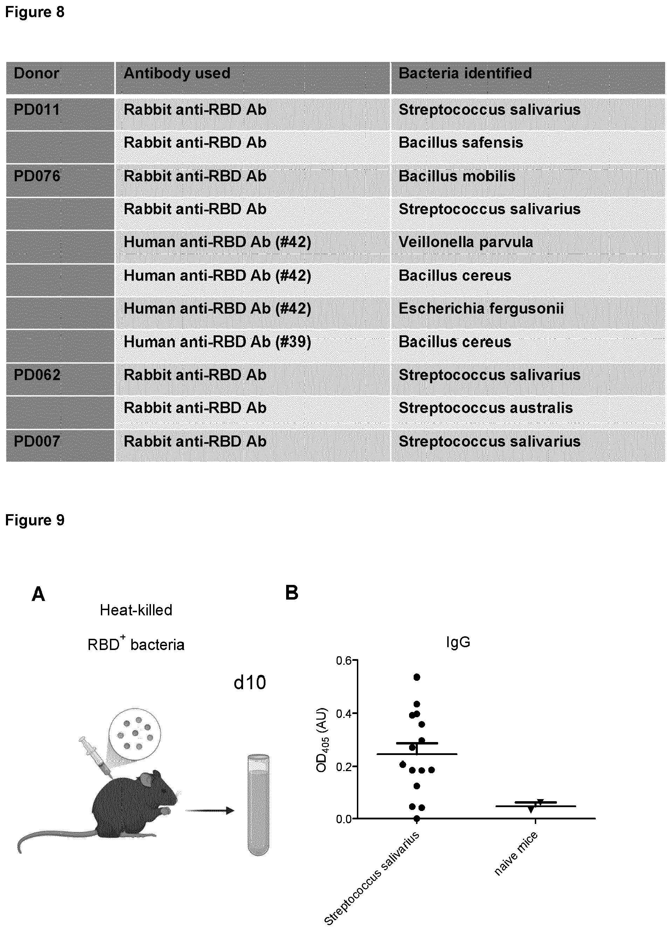

**Fig. 8****: Isolated bacterial strains bound by neutralizing anti-RBD antibodies.**

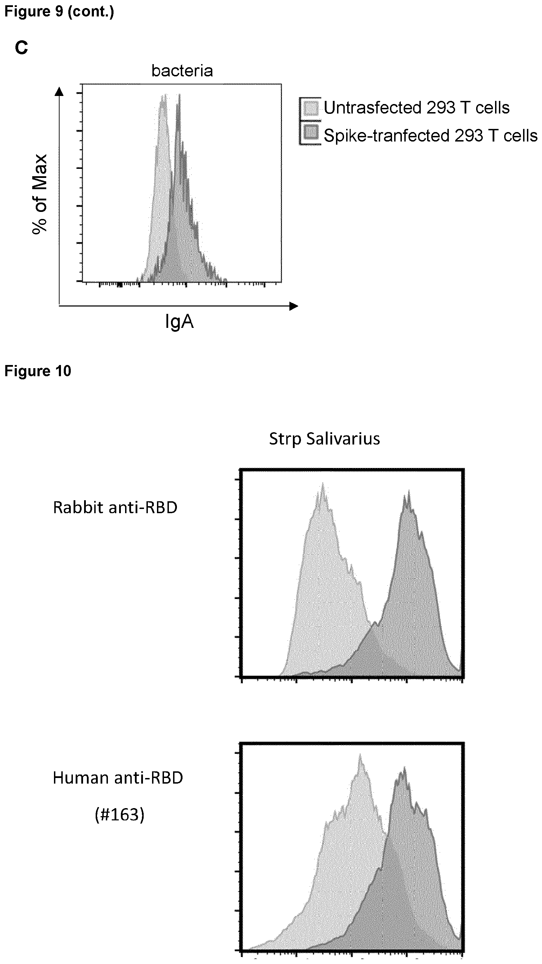

**Fig. 9****: Immunisation with single bacteria strains induced anti-RBD Ig response.** (A) Scheme of the immunization; (B) levels of IgG against RBD of Spike protein of Sars-Cov-2 in mice immunized with Streptococcus salivarius; (C) Binding of IgA antibodies to Spike protein expressed on the surface of 293 T cells.

**Fig. 10****: Commercially available probiotic Streptococcus salivarius K12 is recognized by anti-RBD antibodies.**

### EXAMPLES

The following examples are presented to describe particular embodiments of the invention, without being limiting in scope.

The examples provided herein show the interplay between Sars-CoV-2, the immune system and human commensal microbiota. Here, the contribution of microbiota to the protection of a host against Sars-Cov-2 infection by inducing cross-reactive IgA antibodies against receptor-binding domain (RBD) of Spike protein of Sars-Cov-2 was analyzed. It has been found that anti-RBD binding IgA antibodies are present in Sars-Cov-2-unexposed individuals at their mucosal surfaces. Furthermore, multiple monoclonal neutralizing anti-RBD antibodies isolated from immunized animals and hospitalized COVID-19 patients recognized distinct commensal bacterial species. Some bacterial strains, such as *Streptococcus salivarius,* or *Veillonella parvulla,* are known to be part of normal oral microflora and induced protective anti-RBD IgG response upon immunzation in mice. Finally, severe COVID-19 patients had modified microbiota with outgrowth of Enterococcus and depletion of Streptoccosus genera in their swabs and feces. Thus, these data suggest that distinct microbial species of human microbiota may induce cross-reactive antibodies against RBD and may lower COVID-19 disease severity.

Sars-Cov-2 virus infects cells via interaction of Spike (S) protein with ACE2 receptor expressed by various cells in the body. Spike protein of Sars-Cov-2 contains a receptor-binding domain (RBD) that is particularly important for the infection of cells and blocking of its interaction by monoclonal anti-Sars-Cov-2-RBD antibodies confers protection of the host against infection. Systemic antibodies (mainly of IgG, IgM and IgA1) neutralise virus propagation after successful infection of the host, while the presence of antigen-specific secreted antibodies (IgA2, IgA1 and IgM) at the mucosal surfaces may prevent initial infection of the host. Several studies reported anti-RBD antibody titers in unexposed humans (REFS). The induction of these antibodies was associated with previous infections with other common cold coronaviruses but was not formally shown. Our previous study has highlighted the absence of IgA2 antibodies specific against Sars-Cov-2 antigens in severe COVID-19 patients in the ICU (Gomes M et al., Nat Comms, 2021), suggesting that the presence of IgA antibodies may serve as an underlying mechanism for severe disease.

IgA antibodies at mucosal surfaces are induced upon colonization of the body cavities by commensal microbiota. Apart from induction of IgA, human microbiota at the respiratory airways, digestive tract and on the skin surface influences the fitness of the human immune system. Constant interaction between human microbiota and immune system shapes the immune cell repertoire and fitness of the immune system. It is estimated that human microbiota contains several millions of genes, thus, may serve as a huge reservoir of various antigens. These proteins may partially resemble host proteins, as well as proteins from other microorganisms and may, therefore, mediate cross-reactive immune responses. Such molecular mimicry is suggested to play role as one of the triggers of various autoimmune diseases in genetically susceptible host.

For instance, a link between commensal bacteria antigens and autoimmunity was documented for systemic lupus erythematosus, neuromyelitis optica, myocarditis and multiple sclerosis. Apart from such deleterious functions of microbiota, it may also mediate protection against various infections of bacterial origin, such as Citrobacter rodentium, Clostridium difficiles, Pseudomonas aeruginosa and also of viral origin, like influenza. Such protection may be mediated by regulating the fitness of the innate immune system, like induction of tonic type I IFN production, but also by induction of cross-reactive adaptive antibody responses. Interestingly, cross-reactive antibodies targeting gp41 from HIV-1 have been found to be induced by commensal microbiota in the gut. However, whether induction of cross-reactive antibody responses by microbiota occurs for Sars-Cov-2 remained unknown.

### Example 1: Presence of IgA antibodies reactive against RBD in healthy individuals

To gain insight on the mechanism of appearance of anti-RBD IgA responses, we first analyzed fecal levels of IgA specific to RBD in fecal supernatants of age-matched healthy individuals and severe COVID-19. Consistent with an ongoing immune reaction against virus, severe COVID-19 patients mounted IgA responses against RBD of Spike protein of Sars-Cov-2 (Fig. 1A, B). Next, a proportion of healthy donors also possessed IgA antibodies reactive to RBD in their fecal fluids (Fig. 1A, B). Taking into account that severe COVID-19 cohort and respective healthy controls were quite old, we further expanded our healthy cohort to analyze the presence of anti-RBD IgA with relation to the age (Fig. 1C). Interestingly, we observed that anti-RBD IgA responses in feces correlated with the age of donors (Fig. 1C), suggesting that during ageing such a response is diminished. Given the complexity of fecal material, we next purified IgA antibodies from feces using peptide M agarose and analyzed its binding capacity to RBD. Here again, healthy donors exhibited IgA2 antibodies specific towards RBD, while severe COVID-19 patients largely lack anti-RBD IgA2 in their stools (Fig. 1D).

Since we detected anti-RBD antibodies in healthy humans, we next wondered whether these antibodies might actually inhibit ACE2-RBD interaction. In order to test this, we expressed human ACE2 on 293T cells and incubated ACE2-expressing cells with biotinylated RBD, followed by the detection of bound RBD using streptavidin conjugated with fluorochrome (Fig. 1E-G). Intestinal IgA, as well as salivary IgA (data not shown) inhibited RBD binding to ACE2 (Fig. 1F, G). Thus, healthy individuals possess anti-RBD IgA at the mucosal surfaces that may inhibit its interaction with ACE2.

### Example 2: Neutralizing anti-RBD antibodies recognize commensal bacteria

IgA is induced upon colonization of human cavities by microbiota and was also shown to bind to microbiota (REF), thus we next wondered whether RBD reactive antibodies recognize commensal microbiota (Fig. 2). To do so, we have divided our healthy cohort in two groups based on the presence or absence of anti-RBD IgA in their fecal supernatants: HC RBD-IgA+ and HC RBD-IgA-, respectively. Both donors groups exhibited similar IgA coating of their microbiota (Fig.2A). To gain further insight on the IgA-coated fraction of microbiota, we have sorted IgA-bound bacteria and analysed its bacterial composition by 16S sequencing. Linear discriminant analysis (LDA) combined with effect size (LefSE) algorithm revealed the differences in genera of IgA coated fractions from HC RBD-IgA+ and HC RBD-IgA-. In particular, IgA coated fractions from HC-RBD IgA+ were enriched with Bilophila, Parabacteroides and Vagococcus, while HC RBD-IgA- donors had increased Lachnospira, Pseudomonas and Bacillus genera (Fig. 3B). Altogether, these data further suggested that anti-RBD IgA found in fecal fluids may bind to commensal microbiota.

To address this directly, we have stained human healthy microbiota with monoclonal neutralizing anti-RBD antibodies that were either generated in rabbit by immunization or cloned from hospitalized COVID-19 patients. Monoclonal antibodies from COVID-19 patients have been cloned in the same vector carrying Fc part of human IgG1 and were expressed in 293 T cells, so that did not differ in their glycosylation pattern. Neutralizing anti-RBD antibody generated in rabbit showed binding to a significant proportion of microbiota from HC (Fig. 2C, D). Further staining of microbiota using 15 neutralizing anti-RBD antibodies from hospitalized COVID-19 patients revealed that some of them (HK CV07-287, HL CV07-250) did not bind towards microbiota and others recognised bacteria independent of preexisting intestinal anti-RBD IgA in the fecal fluids (Fig. 2C). Of note, two clonally related antibodies, CV07-200 and CV07- 283, showed similar, but also distinct binding towards human microbiota (Fig. 2D). In line with this, other anti-RBD antibodies recognised similar fecal bacteria populations. Altogether, these data suggested that neutralizing anti-RBD Sars-Cov-2 antibodies recognise distinct commensal bacteria.

### Example 3: Identification of bacteria recognized by neutralizing anti-RBD antibodies

To gain further insight on the bacteria recognised by anti-RBD antibodies, we have stained, sorted and sequenced antibody bound fraction of microbiota from 3 healthy donors using 4 different anti-RBD antibodies (Fig. 3A, B). Further analysis of genera with abundance more than 1% in sorted populations revealed multiple bacterial populations that were bound by antibodies. We noted the difference between identified bacteria among various donors (Fig.3 B), highlighting the interindividual diversity in bacteria composition. As it widely accepted, binding of antibodies to microbiota occurs via specific recognition of antigen or via FC part of the antibody (REFs). Moreover, certain microorganisms of Lachnospricaeae family may bind human VH3 variable regions in non-specific way via production of superantigens. Furthermore, all the antibodies showed reactivity towards Bacteroides, suggesting non-specific binding in this case. At the same time, we noted that some of the antibodies also recognise Clostridia species, Streptococci, Escherichia and Bifidobacteria (Fig. 3B). Finally, among the previously observed genera enriched in IgA bound fraction of HC RBD-IgA+ donors, Parabacteroides and Bilophila were observed to be bound by the anti-RBD antibodies (Fig. 3B). To further interaction between anti-RBD antibodies with microbiota, anti-RBD bound bacterial fraction was sorted into medium and plated on Petri dishes using selective bacterial media. Single bacterial colonies were further expanded and their identity was determined by 16S rRNA Sanger sequencing (Fig. 3C). As a result, several Bacilli species, Streptococci, Staphylococcus epidermidis, Bifidobacteria, several Enterococci species, Vellionella and Acidaminococcus were identified as bacterial strains bound by anti-RBD antibodies (Fig. 3C). Further staining of purified cultures confirmed their recognition by anti-RBD antibodies (Fig. 3D). Since one of the bacterial strains, which was isolated was Streptococcus salivarius, we next also analysed binding of the antibodies towards probiotic strain, BLIS K12. Blis K12 was found to be recognized by rabbit anti-RBD, as well as HL CV07-250 (Fig. 3D and Suppl. Data not shown). Of note, some bacteria cultures showed only partial staining with anti-RBD antibodies, probably reflecting the heterogeneity of bacteria during growth or community-dependent surface variability. Interestingly, binding of rabbit anti-RBD antibody to Bididobacterium pseudocatenulatum was reduced by preincubation with HL CV07-250, suggesting that these antibodies compete for the binding with the antigen (Suppl. Data not shown). To further determined the nature of the antigen expressed by bacteria, bacterial lysates were analysed by Western blotting. Rabbit anti-RBD antibody and HL CV07-250 recognised protein with Mr round 75 kDa from both cloned Streptococcus salivarius, as well as BLIS K12 (Fig. 3E), while the same antibodies were found to bind protein around 60 kDa (Fig. 3F). Of note, rabbit anti-RBD antibody bound to the linear epitope of RBD, while HL CV07-250 was specific to conformational epitope of RBD (Fig. 3F). Subsequent mass-spectroscopy analysis revealed that rabbit anti-RBD antibody was bound elongation factor G (EF-G) from Streptococcus salivarius, while 60 kDa protein from Bidifobacterium pseudocatenulatum was ABC transporter substrate-binding protein (Fig. 3E, F). Thus, these data suggest that neutralizing anti-RBD antibodies may cross-react towards commensal microbiota.

### Example 4: Distinct microbiota species can induce neutralizing anti-RBD response

Given that anti-RBD antibodies may cross-react towards bacterial proteins we next wondered whether these microorganisms may induce cross-reactive anti-RBD antibody response. For this, we immunized mice with heat-killed bacteria and analyzed antibody responses against RBD. Interestingly, mice immunized with Streptococcus developed anti-RBD IgG in their sera (Fig. 4A). At the same time, we failed to detect anti-RBD antibody responses upon immunization with Bifidobacterium pseudocatenulatum (Fig. 4B). Thus, isolated bacterial strains might induce anti-RBD responses.

Given that these bacterial strains were purified using neutralizing anti-RBD antibodies, we next tested whether the antibodies induced in immunized mice can neutralize pseudovirus expressing the Spike protein (Suppl. Data not shown). Indeed, sera from immunized mice were able to inhibit infection of Vero6 cells by pseudovirus (Fig. 4C). Finally, sera from mice immunized with Streptococcus salivarius K12 also inhibited binding of RBD to ACE2 expressed in 293 T cells (Fig. 4D). Thus, identified bacterial strains may induce neutralizing antibody responses against Sars-Cov-2.

### Example 5: Oral microbiota changes during COVID-19

Since the main route of virus entry is via respiratory and intestinal routes, we first analysed oral microbiota composition in healthy donors and patients infected with Sars-Cov-2 and also patients displaying flu-like symptoms, but negative for Sars-Cov-2. Taking into account that the most immunologically relevant bacterial populations are associated with mucosal barriers, we have chosen to analyse swabs as a source of oral microbiota (Fig. 5). First of all, principal component analysis revealed that the oral microbiota of hospitalized severe COVID patients was different from healthy, mild COVID or patients with flu-like illness (Fig. 5A). Swab microbiota from severe COVID-19 was characterized by decreased bacterial diversity (Fig. 5B), suggesting that oral bacterial diversity may be associated with development of severe COVID. Subsequent LefSE analysis have revealed bacterial genera that are associated with inflammation were enhanced in severe COVID (Fig. 5C). In particular, severe COVID-19 patients had increased genera of Enterococcus, Staphylococcus and Escherichia/Shigella in their swabs (Fig. 5C, D). Interestingly, decrease in Veillonella and Streptococcus genera, bacteria strains of which were isolated as potential inducers of cross-reactive antibodies, were significantly reduced during severe COVID (Fig. 5C, D).

### Example 6: Fecal microbiota changes during severe COVID-19

Next, we wondered how these changes in oral microbiota are reflected in the intestinal compartment during severe COVID-19 disease. To analyse this, we have sequenced fecal microbiota from age-matched healthy and severe COVID-19. PCA analysis of microbiota composition revealed significant differences in the microbiota of severe COVID-19 in comparison to healthy controls (Fig. 6A). Microbiota from severe COVID patients had reduced bacterial diversity (Fig. 6B) with dominance of opportunistic bacterial strains, such as Enterococcus, Staphylococcus and Vagococcus (Fig. 6C, D). while depletion of Alistipes and Dialister was observed in severe COVID. Consistently, Streptococcus genera was significantly diminished in ICU patients (Fig. 6C, D). Thus, sever COVID-19 is associated with outgrowth of opportunistic bacteria (Enterococci, Staphylococci), while some genera, like Streptococcus, Veillonella were depleted from the mucosal surfaces.

### Example 7: Streptococcus salivarius has been identified to react with anti-RBD antibodies.

In order to get an insight on the bacteria bound by antibodies against RBD protein, we have sorted bacteria fractions covered by antibodies and determined their origin via 16S RNA sequencing (data in preparation). Next, we have sorted and cultured antibody bound fractions of microflora in anaerobic conditions (Fig. 8). Resulting single colonies were further expanded, tested for their recognition by anti-RBD antibodies and their identity was determined by 16S RNA sequencing. Interestingly, some of the cloned microbiota species are usual inhabitants of oral microflora. Among them, *Streptococcus salivarius* has been identified. Altogether, these data showed that neutralizing anti-RBD antibodies bind to distinct microflora species.

### Example 8: Sera from immunized mice was able to inhibit the infection of Vero6 cells by pseudovirus.

To further test their functional relevance, we immunized mice with heat-killed bacteria and analyzed antibody responses against RBD. Interestingly, immunized mice exhibited anti-RBD IgG and IgA responses in their sera (Fig. 9). Thus, isolated bacterial strains might induce anti-viral responses. To further gain insight on the functionality of these antibodies, sera from immunized mice was further tested on its capacity to neutralize pseudovirus expressing Spike protein. Indeed, sera from immunized mice was able to inhibit infection of Vero6 cells by pseudovirus (Fig. 4B). Thus, identified bacterial strains may induce neutralizing antibody responses against Sars-Cov-2.

### Example 9: Streptococcus salivarius is recognized by neutralizing antibodies.

Some bacterial strains of *Streptococcus salivarius* are currently used as probiotic for the restoration of oral microflora. Thus, we finally tested whether anti-RBD recognize also this clinically approved strain. Indeed, this strain was also recognized by neutralizing antibodies (Fig. 10).

### Summary

Altogether, the data presented here show that defined microbiota species can induce cross-reactive antibody responses that may neutralize virus infection.

Sars-Cov-2 infection of human mucosal surfaces induces an inflammatory syndrome that may progress towards fatal disease. Multiple factors, both host intrinsic and host extrinsic, were uncovered as drivers of disease progression. Host-derived risk factors include presence of autoantibodies against type I IFN, genetic loci and preexisting disease conditions, like type I diabetes, obesity as well as ageing. Furthermore, pre-existing memory T cells recognising Sars-Cov-2 antigens are a potential risk factor during COVID (Gomes, Bacher). In addition to that, here we reported that healthy, unexposed individuals have a preexisting antibody repertoire at mucosal surfaces that can bind to RBD of Sars-Cov-2 and this response is decreased with age. Thus, both arms of adaptive immune responses may contribute to initial protection of the host and are decreased with age.

Apart from host-intrinsic factors, initial virus load may also define disease outcome. At the same time, there is increasing evidence of microbiota changes during severe COVID-19, suggesting that also microbiota composition as one of the risk factors for the development of acute disease. While certain discrepancy in the published findings about connection of microbiota and COVID-19 severity in terms of genera associated with disease severity exists, this may be explained by various cohorts used for the study and treatments used in each study for the patients. Howver, the common trend is that acute COVID-19 is associated with prevalence of opportunistic bacteria and underrepresentation of immunomodulatory bacteria in almost all of these studies. Our study further supports these findings showing that in our patient cohort increase of Enterococci, Staphylococci and Vagococci, while Veillonella and Streptococci were depleted. Thus, it is plausible that severe COVID-19 is propagated in humans by concert action of the virus and proinflammatory microorganisms. Further prospective studies will shed light on the connection of microbiota composition and critical course of COVID-19.

Microbiota may contribute to the protection of the host from infection via modulating ACE2 receptor expression, induction of tonic type I IFN responses, and tuning systemic TGF-β1 levels (Marta et al). Apart from this, several autoimmune diseases were shown to be induced via antigenic mimicry, while the same mechanism for Sars-Cov-2 was not reported. Here we identified bacteria in the gut flora that expose antigens on their surface which mimic the RBD of SARS-Cov-2 to an extent that these were recognized by anti-RBD antibodies of different origin. Notably, the presence of certain microorganism at mucosal surfaces does not necessarily result in the induction of adaptive immune responses: efficient transport of bacterial antigens to gut-associated lymphoid organs should occur to elicit such response. Thus, it is of importance to dissect the mechanisms of efficient presentation of commensal bacteria to the immune system to elicit cross-reactive protective responses.

Modulation of the human gut flora with specific bacterial strains that occur widely and are recognized as commensals represents a novel preventive, therapeutic approach. However, the potential of identified bacteria to induce cross-reactive protective responses in humans is of the great importance. Yet in principle, the setup of this study suggests the potential of gut bacteria to induce antibody responses against any virus or pathogen, which goes in line with the application of Streptococcus salivarius species for the protection and restoration of the oral flora. Thus, our study suggests that certain commensal bacterial may induce neutralizing anti-RBD antibodies, which could be protective in the case of virus entry.

### Materials and methods

### Patient cohort: Human Donors:

The recruitment of study subjects was conducted in accordance with the Ethics Committee of the Charité (EA 1/144/13 with EA 1/075/19 and EA 2/066/20) and was in compliance with the Declaration of Helsinki.

### Stool sample preparation:

Fresh stool samples of patients and healthy controls were stored on ice or at 4 ° C before processing within 48 h. The stool was diluted in autoclaved and sterile-filtered PBS (inhouse, Steritop^{®} Millipore Express^{®}PLUS 0.22 µm, Cat. No: 2GPT05RE) according to weight in the ratio 100 µg/mL and homogenized by vortex and spatula. The feces solution was then subsequently filtered through 70 µm (Falcon, Cat. No. 352350) and 30 µm filters (CellTrics^{®}, Sysmex, Cat. No. 04-0042-2316) and centrifuged at 4000 x g to pellet the bacterial cells. The supernatant of this centrifugation step was once more centrifuged at 13,000 x g to pellet residual cells. The cell free supernatant was filtered through a 0.22 µm syringe top (Filtropur, Sarstedt Cat. No. 83.1826.001) filter and stored at - 80 °C until further use. Pellets of both centrifugation steps were pooled and re-suspended in 10 mL PBS to measure the cell density at 600 nm. For each working stock a cell amount resembling 0.4 OD was stored in 1 mL of a 40 % glycerol in LB medium mixture in Safe Seal 2 mL reaction tubes (Sarstedt, Cat. No. 72.695.500) and transferred to - 80 °C.

### 16S sequencing, Metaaenomics:

For 16 S rDNA sequencing we amplified the V3/V4 region directly form the sorted samples with a prolonged initial heating step as described by "16S Metagenomic Sequencing Library Preparation" for the Illumina MiSeq System. After the amplicon the genomic DNA was removed by AmPure XP Beads (Beckman Coulter Life Science Cat. No. A63881) with a 1:1.25 ratio of sample to beads (v/v). Next the amplicons were checked for their size and purity on an 1.5 % agarose gel and if suitable subjected to the index PCR using the Nextera XT Index Kit v2 Set C (Illumina, FC-131-2003). After index PCR the samples were cleaned again with AmPure XP Beads (Beckman Coulter Life Science Cat. No. A63881) in a 1: 0.8 ratio of sample to beads (v/v). Samples were then analyzed by capillary gel electrophoresis (Agilent Fragment Analyzer 5200) for correct size and purity with the NGS standard sensitivity fragment analysis kit (Agilent Cat. No. DF-473). Of all suitable samples a pool of 2 µM was generated and loaded to the Illumina MySeq 2500 system.

### Bacteria flow cytometry and sorting:

### Microbiota staining:

The frozen microbiota stocks were topped up with 1 mL of autoclaved and sterile-filtered PBS to reduce glycerol toxicity while thawing. Samples were centrifuged at 13,000 xg for 10 min twice, the supernatant removed and the pellets re-suspended in PBS and finally divided into 10 tests. All stainings of microbiota samples were performed in a DNase containing buffer (PBS/ 0.2 % BSA/25 µg/µL DNase, Sigma Aldrich Cat. No. 10104159001). Staining for human immunoglobulins was performed in 100 µL with 1:50 (v/v) of the detection antibodies: anti-human IgM Brilliant Violet 650 (clone: MHM-88, Biolegend^{®} Cat. No. 314526), anti-human IgG PE/ Dazzle^{™} 594(clone: HP6017, Biolegend^{®} Cat. No. 409324), anti-human IgA1 Alexa Fluor 647 (clone: B3506B4, Southern Biotech Cat. No. 9130-31), anti-human IgA2 Alexa Fluor 488 (clone: A9604D2, Southern Biotech Cat. No. 9140-30). The samples were incubated for 30 minnutes at 4 ° C and directly topped up with 1 mL of a 4 µM Hoechst 33342 solution (Thermo Scientific Cat. No. 62249) for another 30 min at 4 °C. For the detection of Spike protein- similar structures the samples were first incubated in 100 µL containing 0,5 µg SARS-Vov-2 Spike Neutralizing Antibody (clone: HA14JL2302, Sino Biological Inc. Cat. No: 40592-R001) for 30 min at 4 °C then washed with 1 mL PBS and stained again in 100 µL containing 1 µg of the anti-Rabbit Alexa 700, which was then topped up with 4 µM Hoechst 33342 solution. After Hoechst 33342 staining 1 mL of PBS was added and the samples centrifuged at 13,000 x g for 5 min. After removal of supernatant, the samples were re-suspended in PBS/ 0.2 % BSA. The samples were transferred to 5 mL round bottom tubes (Falcon, Cat. No. 352063).

### Microbiota Flow Cytometry:

We used a BD Influx^{®} cell sorter for all cytometric investigations of the microbiota samples. The sheath buffer (PBS) for the instrument was autoclaved and sterile filtered (Steritop^{®} Millipore Express^{®}PLUS 0.22 µm, Cat. No: 2GPT05RE) before each fluidics start up. The quality of each acquisition was assured by the alignment of lasers, laser delays and laser intensities by Sphero^{™} Rainbow Particles (BD Biosciences Cat. No. 559123). The Scatter properties were additionally checked by Megamix-Plus FSC beads (BioCytex Cat. No7802). For sorting, the drop delay was determined prior with Accudrop Beads (BD Biosciences Cat. No. 345249). Samples were acquired with an event rate below 15,000 events and sorted with an event rate below 10,000 events. We always recorded 300,000 Hoechst positive events. We sorted up to 100,000 events for metagenomics directly into Protein Low Bind tubes (Eppendorf Cat. No 022431102), spun down the sample at 17,000 x g and replaced residual sorting buffer by DEPC treated water (Invitrogen Cat. No. 46-2224). The samples were stored in approx. 10 µL at -20 °C until further processing. For subsequent cultivation of bacteria after sorting we sorted directly into PYG medium and transferred the cells directly into an anaerobic chamber.

### Bacteria culture:

PYG medium and plates were prepared as described by the DSMZ (German Collection of Microorganisms and Cell Cultures). 300,000 events were sorted into 1 ml of PYG medium and directly transferred to an COY anaerobic chamber. Sorted bacteria were applied on PYG, BHI (Brain heart infusion broth, Sigma, Cat. No. 53286-100G)) and Fastidious agar plates (Thermo Scientific, Cat. No. 12957138) and bacteria was allowed to grow for 24 hours. Colonies were picked and PYG medium, BHI broth and Schaedler broth (Roth, Cat. No. 5772.1) were inoculated with colonies from the respective plates. The next day, DNA was isolated and the remaining bacteria were frozen in 40% glycerol LB medium in liquid nitrogen.

### Enzyme-linked immunosorbent assay:

For the detection of antibody titers in sera and fecal supernatants 96-well plates were coated with goat anti human Ig (H+L chain) antibody (Southern Biotech, Cat. No. 2010-01) or goat anti human IgA (Fa(b)) (Southern Biotech, Cat. No. 2050-01) antibody for the detection of IgG, IgM and IgA respectively. After washing with 1x PBST for 30 second, the plates were blocked with 200 µL of 5% PBS/BSA for 1 hour at room temperature. Next, plates were washed 3 times with 200 µL of 1x PBST for 30 second at a time. The sera and fecal supernatants were diluted in PBS and 100 µL were added to the plate. Standards were diluted in PBS and applied to the plate: IgA1 (Genway, Cat. No. E04696), IgM (Sigma, Cat. No. 18260), IgA2 (Genway, Cat. No. 50D1F7), IgG () then the plates were incubated over night at 4°C. After that, plates were washed 5 times with 200 µL of 1x PBST and detection antibodies were applied: anti-human IgG-AP (ICN/Cappel, Cat No. 59289), anti-human IgM-AP (Sigma, Cat. No. A3437-.25ML), anti-human IgA-AP (Sigma, Cat.No. A2043), anti-human IgA1-AP (SouthernBiotech, clone: B3506B4, Cat. No. 9130-04), anti-human IgA2-AP (SouthernBiotech, clone: A9604D2, Cat. No. 9140-04) and were incubated for 1 hour at 37°C. Subsequently, the plates were washed 5 times with 200 µL of 1x PBST 100 mcl of pNPP (Sigma) was added to each well. Reactions were stopped by addition of 3M NaOH. Optical densities were measured on Spectramax (Molecular devices).

To determine the RBD specific antibody titers, 96-well plates were coated over night with 1 µg/ml recombinant SARS-CoV-2 (2019-nCoV) Spike Protein (RBD, His Tag, Sino biological) protein Plates were washed, blocked and the administration of sera and fecal supernatants were done as previously described. To detect anti RBD specific IgA a biotinylated anti human IgA antibody (Southern Biotech, Cat. No. 2050-08) was applied, followed by an incubation for 1 h at 37°C. After washing 6 times with PBST, streptavidin-HRP (invitrogen) was added and after 1 hour incubation at RT and 5 times washing with PBST, Tetramethylbenzidine (TMB) Substrate (invitrogen) was added. The reaction was stopped by addition of 2N H2SO4. Optical densities were measured on Spectramax (Molecular devices).

### Anti-RBD Enzyme-linked immunosorbent assay:

To determine the RBD specific antibody titers, 96-well plates were coated over night with 1 µg/ml recombinant SARS-CoV-2 (2019-nCoV) Spike Protein (RBD, His Tag, Sino biological) protein Plates were washed, blocked and the administration of sera and fecal supernatants were done as previously described. To detect anti RBD specific IgA a biotinylated anti human IgA antibody (Southern Biotech, Cat. No. 2050-08) was applied, followed by an incubation for 1 h at 37°C. After washing 6 times with PBST, streptavidin-HRP (invitrogen) was added and after 1 hour incubation at RT and 5 times washing with PBST, Tetramethylbenzidine (TMB) Substrate (invitrogen) was added. The reaction was stopped by addition of 2N H2SO4. Optical densities were measured on Spectramax (Molecular devices).

### Flow cytometric assay for analysis of anti-Spike protein responses:

HEK293T cells were transfected with a plasmid expressing wild-type SARS-CoV-2 S protein. Next day, the proportion of transfected cells was determined by staining with anti-SARS-CoV-2 Spike Glycoprotein S1 antibody (clone: CR3022, Abcam, Cat. No. ab273073) for 30 min, wash cells once with PBS/ 0.2 % BSA and subsequent staining with anti-human IgG PE/ Dazzle^{™} 594(clone: HP6017, Biolegend^{®} Cat. No. 409324). Further transfected cells were collected and incubated with sera or fecal supernatants for 30 min, washed twice with PBS/BSA and stained with anti-human IgM Brilliant Violet 650 (clone: MHM-88, Biolegend^{®} Cat. No. 314526), anti-human IgG PE/ Dazzle^{™} 594(clone: HP6017, Biolegend^{®} Cat. No. 409324), anti-human IgA1 Alexa Fluor 647 (clone: B3506B4, Southern Biotech Cat. No. 9130-31), anti-human IgA2 Alexa Fluor 488 (clone: A9604D2, Southern Biotech Cat. No. 9140-30). Cells were washed with PBS/ 0.2 % BSA and either measured directly, dead cell exclusion by DAPI or stained for dead cells with Zombie Violet^{™} (Biolegend^{®} Cat. No. 423113) in PBS for 5 min at room temperature and fixed in 4 % paraformaldehyde solution overnight at 4°C. Samples were acquired on a FACSCanto (BD Biosciences) or a MACS Quant 16 (Miltenyi) and analyzed using FlowJo v10 (Tree Star Inc.) analysis software.

### Flow cytometric assay for analysis of ACE2-RBD interactions:

HEK293T cells were transfected with a plasmid expressing human ACE2 protein. Next day, the proportion of transfected cells was determined by staining with biotinylated RBD (Sino biologicals, Cat: 40592-V08H-B) for 30 min, wash cells once with PBS/ 0.2 % BSA and subsequent staining with streptavidin-FITC (Thermo Fischer Scientific: Cat. No. 11-4317-87). Further transfected cells were collected and incubated with biological samples for 30 min, washed twice with PBS/BSA and with biotinylated RBD (Sino biologicals, Cat: 40592-V08H-B) for 30 min, wash cells once with PBS/ 0.2 % BSA and subsequent staining with streptavidin-FITC (Thermo Fischer Scientific: Cat. No. 11-4317-87). Cells were washed with PBS/ 0.2 % BSA and either measured directly, dead cell exclusion by DAPI or stained for dead cells with Zombie Violet^{™} (Biolegend^{®} Cat. No. 423113) in PBS for 5 min at room temperature and fixed in 4 % paraformaldehyde solution overnight at 4°C. Samples were acquired on a FACSCanto (BD Biosciences) and analyzed using FlowJo v10 (Tree Star Inc.) analysis software.

### Mice immunizations:

Grown bacteria was collected, washed three time with PBS and heat-inactivated at 65 C for 1 hr. Heat inactivated bacteria was resuspended with final OD=1.0. C57 BI/6 mice were injected with 200 mcl of heat-killed bacteria.

### Pseudovirus neutralisation assay:

Pseudovirus neutralization assay for SARS-CoV-2 was performed as previously described. Briefly, 2µL of serum or serum dilutions were pre-incubated for 1 hour at 37°C with 10µL of virus in 10µL PBS. The pre-incubated serum/virus suspensions were then added to VeroE6 cells cultured in 100µL DMEM 5% hiFCS on a 96-well plate, and incubated at 37°C for 1 day before readout (plaques/Fluorescent foci). Assay was performed in duplicate wells, three dilutions per serum. Analysis was done using averages of 4 fields of view chosen randomly (blind) and then assessed for foci using fluorescent microscopes.

### Protein gel electrophoresis and Western blotting:

Overnight bacterial cultures were pelleted and were resuspended in RIPA buffer containing protease inhibitors cocktail (Roche). Samples were sonicated at 50% voltage for 5 cycles of 10 sec pulses followed by 30 sec rest on ice. After sonication glass beads (MP Biomedicals) were added as 1/3 of total volume to the bacterial extract. Samples were vortexed for 30 sec followed by chilling on ice for 30 sec (for a total of 5 cycles). Lysates were spun down for 10 min at 20000g and supernatant was collected. For western blot analysis samples were run on 12% SDS-PAGE under reducing conditions and transferred to PVDF membrane (Bio-Rad). SARS-CoV-2 (2019-nCoV) Spike RBD-His Recombinant Protein (Sino Biological: Cat No:) was used as a positive control. Membrane was blocked by incubation in 5% non-fat milk (Roth) in TBST buffer for 1 h at room temperature with constant shaking. Subsequently membrane was hybridized with rabbit neutralizing anti-RBD antibody (Sino Biological) in blocking solution for 1h at room temperature with constant shaking. Membrane was then washed in TBST and incubated with anti-rabbit IgG-HRP (Cell signaling) for 1 h at room temperature with constant shaking. SuperSignal West Fempto Maximum Sensitivity (Thermo Fisher scientific) substrate kit was used. The signal was acquired using Chemi Doc imaging system (Bio-rad).

### Mass-spectroscopy analysis of proteins:

The protein bands after 1D-PAGE were excised and washed twice with 100 mL of 0.1 M NH4HCO3 (pH 7.5) and 50% acetonitrile mixture at 50 oC until the piece of gel becomes transparent. Protein cysteine bonds were reduced with 10mM DTT in 50 mM NH4HCO3 for 30 min at 56 °C and alkylated with 15 mM iodoacetamide in the dark at RT for 30 min. The step with adding DTT was repeated. Then gel pieces were dehydrated with 100 mcl of acetonitrile, air-dried and treated by 10 mkl of 12 mg/mL solution of trypsin (Trypsin Gold, Mass Spectrometry Grade, Promega) in 50 mM ammonium bicarbonate for 15 h at 37oC. Peptides were extracted with 20 mcl of 0.5% trifluoroacetic acid water solution for 30 min with sonication, dried in a SpeedVac (Labconco) and resuspended in 3% ACN, 0.1% TFA. Aliquots (2 mkl) from the sample were mixed on a steel target with 0.3 mcl of 2,5-dihydroxybenzoic acid (Sigma Aldrich) solution (30 mg in 400mkl of 30% acetonitrile/0.5% trifluoroacetic acid), and the droplet was left to dry at room temperature. Mass spectra were recorded on the Ultraflex II MALDI-ToF-ToF mass spectrometer (Bruker Daltonik, Germany) equipped with an Nd laser. The [MH]+ molecular ions were measured in reflector mode, the accuracy of the mass peak measurement was 0.007%. Fragment ion spectra were generated by laser-induced dissociation, slightly accelerated by low-energy collision-induced dissociation, using helium as a collision gas. The accuracy of the fragment ions mass peak measurement was 1Da. Correspondence of the found MS/MS fragments to the proteins was performed with the help of Biotools software (Bruker Daltonik, Germany) and a Mascot MS/MS ion search.

Cloning and expression of identified proteins is performed as described in the prior art, e.g. in the handbook of Protein Expression Overview from ThermoFisher scientific.

### Linear discriminant analysis effect size (LefSE) of microbiota is performed as described in the prior art.

Linear discriminant analysis effect size (LEfSe) model was used to identify species having different relative abundance between groups. Only the taxa meeting a LDA threshold value of >2 was considered significant. For species identified by LEfSe model, an alternative analysis was performed to confirm the difference of their relative abundance between groups by Wilcoxon Rank Sum test. P-values <0.05 were considered statistically significant.

## Claims

1. An immunogenic composition for use as a medicament to prevent and/or treat a medical condition associated with a SARS-CoV infection, said composition comprising one or more bacteria of the human microbiota and/or immunogenic parts thereof, wherein said composition is capable of inducing acquired immunity to said infection.

2. The immunogenic composition for use according to the preceding claim, wherein the bacteria is commensal bacteria of the human gastrointestinal, oral and/or respiratory microbiota, preferably of the human oral microbiota.

3. The immunogenic composition for use according to the preceding claim, comprising at least one or more of *Streptococcus salivarius, Bacillus sanfensis, Bacillus pumilis, Bacillus mobilis, Baccilus cereus, Velionella parvulla, Staphylococcus epidermidis, Bifidobacterium pseudocatenulatum, Bifidobacterium longum* or an *Escherichia* strain.

4. The immunogenic composition for use according to any one of the preceding claims, wherein the bacteria binds a neutralising antibody that recognises a spike protein of a SARS-CoV, wherein the neutralising antibody is preferably an IgA antibody, more preferably an IgA2 antibody.

5. The immunogenic composition for use according to any one of the preceding claims, wherein the neutralising antibody that recognises a spike protein of a SARS-CoV binds a receptor-binding domain (RBD) sequence according to SEQ ID NO: 1, or to a sequence with at least 70%, preferably at least 80% or 90%, sequence identity to SEQ ID NO: 1 and/or immunogenic fragments thereof.

6. The immunogenic composition for use according to any one of the preceding claims, wherein the bacteria induces production of a human antibody that recognises the spike protein of a SARS-CoV after administration of said bacteria in a human subject, wherein the SARS-CoV is preferably SARS-CoV-2.

7. The immunogenic composition for use according to any one of the preceding claims, wherein the medical condition associated with a SARS-CoV infection is selected from the group consisting of respiratory conditions, such as pneumonia, acute respiratory distress or severe acute respiratory syndrome (SARS), viral sepsis, kidney failure, liver failure, disseminated intravascular coagulation (DIC) and pathologic cytokine release syndrome.

8. The immunogenic composition for use according to any one of the preceding claims, wherein the bacteria have been prepared for administration to a human subject, wherein the bacteria preferably have been prepared as a vaccine, such as being killed and/or attenuated, for example by treatment with heat.

9. The immunogenic composition for use according to any one of the preceding claims, wherein the composition is configured for induction of an immune response in the mucosa of a subject, preferably wherein the composition is configured for administration in the oral cavity and/or in the respiratory system of a human subject.

10. Isolated bacteria of the human microbiota that bind to a neutralising antibody that recognises the spike protein of SARS-CoV, preferably the spike protein of SARS-CoV2.

11. The isolated bacteria according to claim 10, wherein the bacteria is commensal bacteria of the human gastrointestinal and/or respiratory microbiota, wherein the bacteria is preferably of the human oral microbiota.

12. The isolated bacteria according to any one of claims 10 to 11, wherein said bacteria comprise one or more antigens with structural similarity to SEQ ID NO: 1, and/or an immunogenic fragment thereof, wherein said structural similarity is sufficient to induce production of an antibody in a human subject that binds both the bacterial antigen and a protein according to SEQ ID NO: 1.

13. The isolated bacteria according to any one of claims 10 to 12, comprising at least one of *Streptococcus salivarius, Bacillus sanfensis, Bacillus pumilis, Bacillus mobilis, Baccilus cereus, Velionella parvulla, Staphylococcus epidermidis, Bifidobacterium pseudocatenulatum, Bifidobacterium longum* and an *Escherichia coli* strain.

14. An in vitro method for the diagnosis, prognosis, risk stratification and/or therapy management of a human subject with an increased risk of an adverse event associated with a medical condition associated with a SARS-CoV infection (preferably with SARS-CoV-2), comprising:
a. Providing a sample from the subject,
b. Determining the presence and/or absence and/or an amount of one or more bacteria of the human microbiota (gastrointestinal and/or respiratory, preferably oral microbiota) and/or antibodies thereto in said sample, wherein the bacteria binds a neutralising antibody that recognises a spike protein of SARS-CoV (preferably of SARS-CoV-2), wherein
c. the presence and/or absence and/or amount of said bacteria and/or antibodies thereto indicates a likelihood of an adverse event associated with a medical condition associated with a SARS-CoV infection (preferably with SARS-CoV-2).

15. The method according to the preceding claim, wherein the absence and/or a low amount of said one or more bacteria and/or antibodies thereto (preferably in comparison to a suitable control, such as from a healthy subject and/or a subject with a SARS-CoV infection but without an elevated risk), indicates an elevated risk of an adverse event, and preferably indicates hospital admission.


SEQUENCE LISTING

<110>  Deutsches Rheuma-Forschungszentrum Berlin

<120>  IMMUNOGENIC COMPOSITIONS FOR PRODUCING NEUTRALISING ANTIBODIES
       AGAINST SARS-COV

<130>  XII 2398/20EP2

<150>  EP20211707.3
<151>  2020-12-03

<160>  1

<170>  PatentIn version 3.5

<210>  1
<211>  223
<212>  PRT
<213>  viral

<400>  1

Arg Val Gln Pro Thr Glu Ser Ile Val Arg Phe Pro Asn Ile Thr Asn
1               5                   10                  15

Leu Cys Pro Phe Gly Glu Val Phe Asn Ala Thr Arg Phe Ala Ser Val
            20                  25                  30

Tyr Ala Trp Asn Arg Lys Arg Ile Ser Asn Cys Val Ala Asp Tyr Ser
        35                  40                  45

Val Leu Tyr Asn Ser Ala Ser Phe Ser Thr Phe Lys Cys Tyr Gly Val
    50                  55                  60

Ser Pro Thr Lys Leu Asn Asp Leu Cys Phe Thr Asn Val Tyr Ala Asp
65                  70                  75                  80

Ser Phe Val Ile Arg Gly Asp Glu Val Arg Gln Ile Ala Pro Gly Gln
                85                  90                  95

Thr Gly Lys Ile Ala Asp Tyr Asn Tyr Lys Leu Pro Asp Asp Phe Thr
            100                 105                 110

Gly Cys Val Ile Ala Trp Asn Ser Asn Asn Leu Asp Ser Lys Val Gly
        115                 120                 125

Gly Asn Tyr Asn Tyr Leu Tyr Arg Leu Phe Arg Lys Ser Asn Leu Lys
    130                 135                 140

Pro Phe Glu Arg Asp Ile Ser Thr Glu Ile Tyr Gln Ala Gly Ser Thr
145                 150                 155                 160

Pro Cys Asn Gly Val Glu Gly Phe Asn Cys Tyr Phe Pro Leu Gln Ser
                165                 170                 175

Tyr Gly Phe Gln Pro Thr Asn Gly Val Gly Tyr Gln Pro Tyr Arg Val
            180                 185                 190

Val Val Leu Ser Phe Glu Leu Leu His Ala Pro Ala Thr Val Cys Gly
        195                 200                 205

Pro Lys Lys Ser Thr Asn Leu Val Lys Asn Lys Cys Val Asn Phe
    210                 215                 220